# EUROPEAN PATENT SPECIFICATION

(11) **EP 4 100 723 B1**
(45) Date of publication and mention of the grant of the patent: **19.07.2023**
(21) Application number: 22704777.6
(22) Date of filing: 11.02.2022
(51) Int. Cl.: G01N 21/65, A61B 1/07, A61B 5/00

(54) **RAMAN SPECTROSCOPY PROBE, RAMAN SPECTROSCOPY APPARATUS INCLUDING THE RAMAN SPECTROSCOPY PROBE AND ELONGATE ASSEMBLY**
MESSSONDE FÜR RAMAN SPEKTROSKOPIE, VORRICHTUNG EINSCHLIESSLICH DER MESSSONDE FÜR RAMAN SPEKTROSKOPIE UND LANGGESTRECKTE ANORDNUNG
SONDE DE SPECTROSCOPIE RAMAN, APPAREIL DE SPECTROSCOPIE RAMAN COMPRENANT LA SONDE DE SPECTROSCOPIE RAMAN ET ENSEMBLE ALLONGÉ

(30) Priority: 12.02.2021 GB 202101994
(43) Date of publication of application: 14.12.2022
(73) Proprietor: Creo Medical Limited, Chepstow, Monmouthshire NP16 5UH (GB)
(72) Inventor: HANCOCK, Christopher Paul, Chepstow NP16 5UH (GB)
(74) Representative: Mewburn Ellis LLP
(86) International application number: PCT/EP2022/053420
(87) International publication number: WO 2022/171824

(56) References cited:
- CN-B- 108 066 001
- JP-A- 2015 109 982
- US-A1- 2006 217 594
- US-A1- 2007 038 123
- US-A1- 2011 190 760
- US-A1- 2014 316 255

## Description

### TECHNICAL FIELD

The invention also refers to a Raman spectroscopy apparatus including the Raman spectroscopy probe and an analysis device. The Raman spectroscopy probe has a lumen configured to receive an elongate instrument (e.g. a camera, a surgical instrument and/or an electrosurgical treatment device for delivering electromagnetic radiation for tissue treatment), and at least one Raman fibre arranged outside the lumen.

### BACKGROUND

Raman spectroscopy can be used to identify different types of tissue of a human being or a mammal. In addition, it has been demonstrated that Raman spectroscopy can be used to identify cancerous tissue or other forms of tissue degeneration. This capability of Raman spectroscopy can also be used in endoscopic treatment of tissue for example for identifying the tissue to be treated.

US 8,175,423 and US 8,702,321 disclose an endoscopic Raman probe including a plurality of optical fibres for collecting Raman scattered light.

Direct vision either using CMOS or CCD cells, with illumination from LEDs or Halogen bulbs is used in endoscopes to diagnose diseased tissue by the eye is commonly used in endoscopy and bronchoscopy suites.

US 2007/038123 A1 discloses a Raman artery probe diagnostic system and a method of using the system for the diagnosis of vascular disease. A side looking catheter having light delivery and collection optical fibers can deliver infrared light in a radial direction from the catheter to diagnose atherosclerotic plaques in the coronary arteries. US 2006/217594 A1 refers to an endoscope for in vivo imaging the cells, tissue, organs or body cavities of humans or other animals to observe and locate, diagnosis and/or treat disease. Illumination sources, image detectors, sensors may be provided alone or in combination on the removable tip allowing functional alterations or optimization for a particular procedure.

### SUMMARY

The invention is defined by the independent claims. The dependent claims describe preferred embodiments of the invention.

A Raman spectroscopy probe comprises an elongate body having a distal end and a proximal end, at least one Raman fibre, and an instrument lumen. The at least one Raman fibre is arranged within the elongate body for guiding light between the proximal end and the distal end. The instrument lumen is arranged within the elongate body and extending between the distal end and the proximal end. The instrument lumen is configured to receive an elongate instrument, for example the instrument lumen may function as an endoscopic instrument/operating/working channel. The at least one Raman fibre is arranged outside of the instrument lumen.

A Raman spectroscopy apparatus (or Raman spectroscopy unit) comprises the Raman spectroscopy probe described above and an analysis device including a Raman light source for generating monochromatic light (such as a laser that lases at a wavelength in the EM spectrum between infrared and ultraviolet light) and a spectrometer, wherein the at least one Raman fibre is connected to (e.g. in optical communication with) the analysis device, in particular connected to (e.g. in optical communication with) the monochromatic light source and/or the spectrometer. In a particular arrangement, the illumination fibre may also be used to deliver light at a single a single wavelength or a plurality of wavelengths in the visible region to illuminate the tissue so that a CMOS (or CCD) sensor/array of sensors can be used to visualise the target tissue and any defects that can be seen by the eye.

The terms "connected" and "coupled" are used herein in an open sense, in that, when one element is said to be connected/coupled to another element, it is to be understood that the two elements may be directly connected/coupled together such that there are no intervening elements and also that the two elements may be indirectly connected/coupled together such that there are one or more intervening elements. However, when two elements are said to be "directly connected" or "directly coupled" together, it is to be understood that there are no intervening elements between them.

Raman spectroscopy when used with a scoping device (e.g. an endoscope or the like) needs to ensure that sufficient Raman scattered light is collected at the distal and of the scoping device. This may be achieved by providing a large cross-sectional area of the at least one Raman fibre compared with the cross-sectional area of the scoping device. By arranging the at least one Raman fibre outside of the instrument lumen and, thus, optionally on or near to the perimeter of the scoping device, the cross-sectional area of the at least one Raman fibre can be made large while maintaining the diameter of the instrument lumen and of the scoping device. The larger the diameter of the collection fibre and/or the greater the number of collection fibres the better as this will enable as much scattered light as possible to be collected, which will lead to the highest possible electrical signal will be collected for processing once the collected photons are turned into electrons.

Raman spectroscopy is a spectroscopic technique that can be used to analyse tissue by determining vibrational modes of molecules, although rotational and other lowfrequency modes of systems may also be observed when incident EM energy is in the far infrared or even microwave/millimetre wave region. Raman spectroscopy provides a structural fingerprint by which molecules can be identified which then can be associated with a certain type of tissue and/or certain type of degeneration of tissue, for example exhibited by cancerous tissue.

Raman spectroscopy relies upon inelastic scattering of photons, known as Raman scattering. A source of monochromatic light, usually from a laser in the visible, near infrared, or near ultraviolet range is used. It may also be preferable to use longer wavelength light in the mid or far infrared region. The laser light interacts with molecular vibrations, phonons or other excitations in the tissue, resulting in the energy of the laser photons being shifted up or down. The shift in energy gives information about the vibrational modes in the system.

The tissue to be analysed is illuminated with the monochromatic light which is transmitted by the at least one Raman fibre. Electromagnetic radiation from the illuminated tissue is collected and sent to an analysing device via the at least one Raman fibre. A monochromator may be provided in the analysing device. Elastic scattered radiation at the wavelength corresponding to the laser line (Rayleigh scattering) is filtered out by either a notch filter, edge pass filter, or a band pass filter, while the rest of the collected light is dispersed onto a detector of the analysing device.

The at least one Raman fibre may include at least one collection fibre for guiding light from the distal end to the proximal end and/or at least one illumination fibre for guiding light from the proximal end to the distal end. The illumination fibre may also be used to deliver the wideband white light or narrow band light for direct vision as well as Raman spectroscopy. In this arrangement, an optical switch or an optical coupler may be used to deliver either the monochromatic laser light for Raman or the wideband light from say a halogen light bulb for direct vision. Details will be described later.

The Raman spectroscopy probe may be connected to the analysis device. The analysis device may include a monochromatic Raman light source, such as one or more lasers, for illuminating the tissue to be analysed. The Raman light source may be connected to (e.g. in optical communication with) the at least one Raman fibre, in particular connected to (e.g. in optical communication with) the at least one illumination fibre. The analysis device may also include components for analysing the Raman scattered light emitted by the tissue which was subjected to the illumination with the light emitted by the Raman light source. For example, the analysis device may include filters, such as a notch filter, edge pass filter, or a band pass filter, for filtering the Rayleigh scattered light from the light feedback to the analysis device via the at least one collection fibre. The analysis device may include a spectrometer for determining the intensity and/or the wavelength of the Raman scattered light. The spectrometer may include means for dispersing the light to be analysed and an optical detector for detecting the light, such as CCD detector. The means for dispersing the light to be analysed may spatially separate light depending on the wavelength and may include a grating.

The at least one collection fibre may be coupled to (e.g. in optical communication with) the spectrometer and/or the at least one illumination fibre is coupled to (e.g. in optical communication with) the Raman laser light source.

The Raman spectroscopy probe may be used as a scoping device or in conjunction with a scoping device. For example, the Raman spectroscopy probe may be inserted into a catheter or other type of scoping device such as an endoscope, bronchoscope, cystoscope, nephroscope, arthoscope, colonoscope, or laparoscope. In addition, the Raman spectroscopy probe may be used as an endoscope, bronchoscope, cystoscope, nephroscope, arthoscope, colonoscope, or laparoscope.

In addition, the Raman spectroscopy probe may be integrated with a direct vision system comprising a miniature, e.g. 0.65mmx0.65mm, CMOS sensor array and a white light source. In this configuration, the same fibres used for the white light source may also be used to deliver the laser light to the target tissue for Raman spectroscopy. It may also be desirable to introduce the integrated Raman and direct vision probe into a steerable catheter to replace the use of the endoscope or the bronchoscope. For example, a 3mm steerable catheter with a 1.8mm diameter insertion tube may be used to steer the Raman spectroscopy probe into the bronchus and the bronchial tree, e.g. to the 6^{th} or 7^{th} branch of the tree, and also allow the insertion of a flexible microwave energy therapeutic device and/or the integrated Raman/direct vision system probe and/or a separate Raman probe and a direct vision system probe with illumination fibres and the CMOS/CCD sensor array. The Raman spectroscopy probe may be an endoscopic instrument or part of an endoscopic instrument.

The elongate body of the Raman spectroscopy probe may define the outer dimensions of that part of the Raman spectroscopy probe that is inserted into a cavity of a human being or a mammal, a catheter or other type of scoping device. The elongate body may provide the endoscopic capabilities of the Raman spectroscopy probe.

The elongate body and all components arranged within the elongate body may be flexible enough to be guided through an elongated cavity of the human being or the mammal to be treated. For example, this elongated cavity of the human body may be the gastrointestinal tract or the bronchial tree of the lungs that contains bends and twists. In other words, the elongate body and all components arranged within the elongate body may be flexible in order to permit the distal end of the Raman spectroscopy probe, which may also contain a miniature CMOS camera and multiple fibre rings, to flex as it moves through the body to a target area (target site) of a body of a human being or a mammal. The target site maybe a cavity (or zone or region) or part of a cavity (or zone or region) in the body which is to be analysed, treated and/or imaged.

Furthermore, the outer surface of the elongate body may be made from such a material such that it can be sterilised or subjected to a cleaning routines necessary for surgical procedures.

The Raman spectroscopy probe has an elongated structure which can be (solely) constituted by the elongate body. The distal end of the elongate body is that end of the elongated structure of the Raman spectroscopy probe which is to be inserted into a patient. The proximal end of the elongate body is preferably connected to the analysis device or other devices to which the elongate instrument can be connected to, and may in use remain outside the patient. The elongate body may define the distal end to the proximal end of the Raman spectroscopy probe.

The Raman spectroscopy probe may have a rod-like structure. The Raman spectroscopy probe is flexible perpendicular to its direction of extension (corresponding to a longitudinal direction) and rigid in its directions of extension.

The elongate body may have a tubular structure and may include a sheath for insulating or shielding (e.g. protecting) other components of the Raman spectroscopy probe from the outside, such as fluids. The sheath may be a lubricious polymer sheath for reducing friction, for example when inserted into a scoping device and/or with surrounding tissue.

The elongate body may further comprise a strain relief layer to limit the bending of the at least Raman fibre. The strain relief layer may be covered by the sheath described above. The strain relief layer could include a braid, a coil, and/or a tube made of a polymer or metal wire.

The instrument lumen is a passageway arranged within the elongate body of the Raman spectroscopy probe. The instrument lumen preferably completely extends from the distal end to the proximal end. At least a part of the elongate instrument can be arranged or placed within the instrument lumen. Due to the elongated structure of the elongate body and, thus, of the instrument lumen, an elongated part of the elongate instrument can be placed or arranged within the instrument lumen. For example, a distal end of the elongate instrument coincides or is arranged near a region at the distal end of the elongate body. A proximal end of the elongate instrument may extend beyond the proximal end of the elongate body.

The instrument lumen may be defined by a wall or surface which surrounds the instrument lumen from the distal end to the proximal end. The instrument lumen may be separated from the remaining space within the elongate body in a fluid tight manner. The wall may be constituted by a hollow tube arranged within the elongate body.

It is also possible that the instrument lumen is defined by the components arranged within the elongate body such as the at least one illumination fibre, the at least one collection fibre and a filler material placed between the fibres. In this case, no separate wall may be present for providing the instrument lumen.

The Raman fibre is an optical fibre, such as a single-mode fibre or a multi-mode fibre. The at least one illumination fibre may be called an excitation fibre and is configured to transmit light, in particular laser light, from the proximal end to the distal end. Optionally, the at least one illumination fibre extends beyond the proximal end of the elongate body and is connected to a Raman light source such as a laser.

The Raman light source (or excitation light source) may be configured to generate monochromatic light having a wavelength/frequency which coincides with the excitation frequency of the molecule to be analysed using Raman spectroscopy. The monochromatic light may be light having a single wavelength or the wavelength range is narrow. For example, the wavelength is in the visible spectrum, such as 785 nm with 100 mW of power. However, longer wavelengths (lower frequencies) down to far infrared frequencies that also sit in the THZ or mm-wave band may be used. Far infrared (FIR) is a region in the infrared spectrum of electromagnetic radiation. Far infrared is often defined as any radiation with a wavelength of 15 micrometres (µm) to 1 mm (corresponding to a range of about 20 THz to 300 GHz).

The distal end of the at least one illumination fibre may be treated in such a way that light can be emitted from the distal end of the at least one illumination fibre. For example, the distal end surface of the at least one illumination fibre may be inclined to the extension of directions of extension. The angle of inclination may be 90° or less. Alternatively or additionally, the distal end surface of the at least one illumination fibre may be connected to a lens structure. Both options may be provided for directing the light emitted from the at least one illumination fibre to a part of the target area.

Optionally, the at least one illumination fibre may be subjected to a surface treatment such as the application of a coating to reduce backscattering.

The at least one collection fibre is configured to transmit light, in particular Raman scattered light from the target site, from the distal end to the proximal end. The distal end of the at least one collection fibre may be treated in such a way that light can be collected, i.e. coupled into the distal end of the at least one collection fibre. For example, the distal end surface of the at least one collection fibre may be inclined to the extension of directions of extension. The angle of inclination may be 90° or less. Alternatively or additionally, the distal end surface of the at least one collection fibre may be connected to a lens structure. Both options may be provided for collecting as much as Raman scattered light from the illuminated part of the target area and for coupling the Raman scattered light into the at least one collection fibre. The at least one collection fibre may be connected to the analysis device, in particular to the spectrometer.

Optionally, a plurality of Raman fibres, in particular a plurality of illumination fibres and/or a plurality of collection fibres, may be provided. The at least one collection fibre and at least one illumination fibre may be fixed to each other. For example, the space between the at least one illumination fibre and the at least one collection fibre may be filled with an adhesive and/or other filler material.

The at least one Raman fibre may be arranged between the instrument lumen and the elongate body. In other words, the at least one Raman fibre may be arranged between the outer surface of the instrument lumen and an inner surface of the elongate body. For example, the at least one Raman fibre may be arranged in a space defined by the wall surrounding the instrument lumen and the elongate body.

The arrangement of the plurality of Raman fibres may be such that the space defined by the plurality of Raman fibres is not arranged side-by-side to the instrument lumen in a cross-sectional view.

Optionally, at least a part of the plurality of Raman fibres is in contact with the instrument lumen. Thus, the remaining Raman fibres are arranged between the elongate body and a plurality of Raman fibres in contact with the instrument lumen. For example, in a cross-sectional view, the plurality of Raman fibres may be arranged in one or more sections which are arranged between the instrument lumen and the elongate body. In particular, one or more sections follow the shape of the instrument lumen. In other words, in a cross-sectional view, the outer shape of the one or more sections is aligned with the shape of the instrument lumen (e.g. each section may be a circumferential section and may have a generally arc-shaped form).

Optionally, the Raman spectroscopy probe may include a second lumen extending between the distal end and the proximal end. The at least one Raman fibre may be arranged within the second lumen. Preferably, in a cross-sectional view of the elongate body, a section of an outer surface of the second lumen which is closest to a section of an outer surface of the instrument lumen extends parallel to the section of the outer surface of the instrument lumen. That is, a cross-sectional form of the two sections may be better diagrammatically represented as "((" or "))" than as "()" or ")(".

The second lumen may be provided by a further wall or surface which is arranged within the elongate body and extends from the distal end to the proximal end. A section of the outer surface of the wall of the second lumen that is closest to the wall of the instrument lumen may extend parallel (i.e. having a constant distance along the section of the outer surface of the wall of the second lumen) to the corresponding section of the outer surface of the instrument lumen. Thus, the outer surface of the second lumen in which the at least one Raman fibre is arranged may follow the outer surface of the instrument lumen. For example, the outer surface of the instrument lumen and the outer surface of the second lumen have to same curvature at corresponding sections thereof.

The second lumen may be spaced apart from the instrument lumen. Alternatively, the second lumen is in direct contact with the instrument lumen. The instrument lumen may be separated from the second lumen by the wall or hollow tube. Thus, the wall or hollow tube delimits both the instrument lumen and the second lumen.

The plurality of Raman fibres may be arranged in such a way that they are in contact with the instrument lumen. For example, the plurality of illumination fibres and collection fibres may define one or more sections (e.g. circumferential sections) of the surface of the instrument lumen in a cross-sectional view of the elongate body. What is more, the plurality of Raman fibres may completely fill a sector between the instrument lumen and elongate body. For example, the sector is defined by a part of the outer surface of the instrument lumen, a part of the inner surface of the elongate body, and lines connecting the ends of the part outer surface of the instrument lumen with the ends of the part of the inner surface of the elongate body.

Optionally, the instrument lumen may be completely defined by the arrangement of the plurality of Raman fibres. Alternatively, only sections of the surface of the instrument lumen may be defined by the plurality of the Raman fibres. The plurality of Raman fibres may define two or more sections of the surface of the instrument lumen. For example, two opposing sections of the instrument lumen may be defined by the plurality of Raman fibres. The remaining sections of the surface of the instrument lumen may be defined by a filler material arranged within the elongate body. For example, when the elongate body and instrument lumen have a substantially circular cross section, each sector is a different arc-shaped portion, for example, to use a clock face analogy, a first section may define an arc between 12 o'clock and 3 o'clock, and a second section may define an arc between 3 o'clock and 12 o'clock. Alternatively, a first section may define an arc between 1 o'clock and 5 o'clock, and a second section may define an arc between 7 o'clock and 11 o'clock, and filler material may be arranged between 11 o'clock and 1 o'clock, and between 5 o'clock and 7 o'clock.

In an optional embodiment, the Raman spectroscopy probe further comprises a plurality of Raman fibres, in particular a plurality of illumination fibres and collection fibres, wherein, in a cross-sectional view of the elongate body, the plurality of Raman fibres is distributed around the circumference of the instrument lumen, wherein preferably the plurality of Raman fibres is distributed around the majority or entirety of the circumference of the instrument lumen and, wherein further preferably the plurality of Raman fibres forms a ring of fibres.

The plurality of illumination fibres and collective fibres may be distributed around the circumference of the instrument lumen in such a way that the illumination fibres and collection fibres do not contact each other. The space between the illumination fibres and collection fibres may be filled by an adhesive or a filler material which may contribute to the definition of the instrument lumen. Alternatively, the instrument lumen is defined by a wall whereby the plurality of illumination fibres and collection fibres may be in contact with the wall. In these cases, further illumination fibres and/or collection fibres may be provided which are spaced from the instrument lumen by the plurality of collection fibres and illumination fibres described above and defining or participating in the definition of the instrument lumen.

The plurality of illumination fibres and collection fibres may be arranged in one or more sections around the circumference of the instrument lumen or can completely surround the circumference of the instrument lumen. As discussed, the plurality of illumination fibres and collection fibres may be tightly packed such that they contact each other or can be spaced apart from each other, for example by a filler material.

The arrangement not in contact with each other may reduce cross-coupling of light between the individual fibres. However, it is possible that the plurality of Raman fibres may be covered by an optically non-transparent material in order to reduce or eliminate cross-coupling of light between individual fibres. In this case, the plurality of illumination fibres and collection fibres may be in contact with each other, in particular the optically non-transparent cover materials of the Raman fibres contact each other.

In a cross-sectional view, a line intersecting the plurality of illumination fibres and collection fibres may extend around the circumference of the instrument lumen, either completely or at least a section thereof. The line intersecting the plurality of illumination and collection fibres may not intersect with the instrument lumen in a cross-sectional view.

The elongate body and/or the instrument lumen may have a circular, rectangular, oval or ellipsoid shape in a cross-sectional view. The plurality of illumination fibres and collection fibres which are arranged in contact with the instrument lumen may follow the circular, rectangular, oval or ellipsoid shape of the instrument lumen such that at least a part of the plurality of illumination fibres and collection fibres are arranged in a circular, rectangular, oval or ellipsoid shape in a cross-sectional view of the elongate body. Thus, at least a part of the plurality of illumination fibres and collection fibres are arranged parallel to the shape of the instrument lumen in a in a cross-sectional view of the elongate body.

The arrangement of the plurality of illumination fibres and collection fibres in form of a ring may be constituted in that the plurality of collection fibres and illumination fibres contact each other along the circumference of the instrument lumen. Depending on the shape of the instrument lumen in a cross-sectional view, the ring of collection fibres and illumination fibres may have a shape in a cross-sectional view which is identical to the shape of the instrument lumen. Preferably, the instrument lumen has a circular shape in a cross-sectional and the ring of collection fibres and/or illumination fibres may be circular in a cross-sectional view.

The provision of the plurality of illumination fibres and collection fibres around the circumference of the instrument lumen helps to reduce a cross-sectional area of the elongate body in an advantageous manner: the plurality of illumination fibres and collection fibres are arranged close to the perimeter of the elongate body providing sufficient space for arranging a high number of collection fibres and illumination fibres since the plurality of fibres are arranged radially outside. The instrument lumen is arranged radially inside. In this way, for a given diameter of the radius of the elongate body and of the instrument lumen, the space is advantageously allocated compared to a way where the instrument lumen and the plurality of collection fibres and illumination fibres are arranged side-by-side.

In an optional embodiment, the Raman spectroscopy probe further comprises a plurality of Raman fibres which includes a plurality of illumination fibres and a plurality of collection fibres, wherein, in a cross-sectional view of the elongate body, the plurality of illumination fibres are arranged in a first ring of fibres and a plurality of collection fibres are arranged in a second ring of fibres coaxially arranged with the first ring of fibres.

The second ring of collection fibres may surround the first ring of illumination fibres; i.e. the plurality of collection fibres are arranged radially outside of the plurality of illumination fibres. In this way, more collection fibres may be provided compared to the number of illumination fibres. As stated previously, more Raman scattered light can be collected if the number of collection fibres is increased. Thus, this arrangement provides a tightly packed arrangement of collection fibres and illumination fibres resulting in a high number of illumination as well as collection fibres. What is more, the illumination fibres and collection fibres may be easily arranged within the elongate body. For example, the ring of illumination fibres is provided and then, subsequently, the ring of collection fibres.

The plurality of illumination fibres and collection fibres may be in contact with each other. The first ring of illumination fibres may be separated from the second ring of collection fibres by a middle wall or another structural component arranged within the elongate body. However, it is also possible that first ring of illumination fibres may be in contact with the second ring of collection fibres.

The instrument lumen may be arranged within the first ring of illumination fibres. In a cross-sectional view of the elongate body, the instrument lumen, the first ring of illumination fibres, the second ring of collection fibres, and/or the elongate body may be coaxially arranged.

In an optional embodiment, the Raman spectroscopy probe further comprises a plurality of Raman fibres which includes a plurality of illumination fibres and a plurality of collection fibres, wherein, in a cross-sectional view of the elongate body, the illumination fibres and the collection fibres are alternatingly arranged in a ring of fibres or a group of the plurality of collection fibres is arranged in a first section of the single ring and a group of the plurality of illumination fibres arranged in a second section of the single ring. As mentioned previously, it may be desirable to have more collection fibres than illumination fibres and so, then alternatingly arranged, a smaller group of illumination fibres (e.g. 1 or 2) may alternate with a larger group of collection fibres (e.g. 4 or 6). Also, when not alternatingly arranged, the group of illumination fibres (e.g. located in a smaller section between 1 o'clock and 3 o'clock) may include fewer fibres than the group of collection fibres (e.g. located in a larger section between 3 o'clock and 1 o'clock via 12 o'clock).

It is possible that one or more further single rings of illumination fibres and/or collection fibres may be provided whereby each one of the single rings has the above described characteristics. As discussed above, the plurality of illumination fibres and collection fibres may be arranged such that they contact each other or are spaced apart with respect to each other.

Optionally, the first section of the collection fibres is larger than the second section of the illumination fibres. For example, the first section of collection fibres includes two thirds or three quarters of the single ring, while the second section of the illumination fibres includes one third or a quarter of the single ring.

In optional embodiment, the Raman spectroscopy probe further comprises a plurality of Raman fibres which includes a plurality of collection fibres and at least one illumination fibre, wherein, in a cross-sectional view of the body, the plurality of collection fibres is arranged in a single ring of fibres, wherein preferably the at least one illumination fibres is arranged radially outside radially or inside the single ring of collection fibres.

Preferably, two or more illumination fibres are arranged radially inside or radially outside the single ring of illumination fibres. For example, the two or more illumination fibres are arranged in a section along the single ring of collection fibres or are evenly distributed along the single ring of collection fibres.

In optional embodiment, a longitudinal axis of the elongate body is coaxial with a longitudinal axis of the instrument lumen.

In a cross-sectional view of the elongate body, a centre of the elongate body is coaxially arranged with a centre of the instrument lumen. In addition, the centre of the elongate body or the centre of the instrument lumen may be coaxially arranged to the ring or rings of fibres.

The longitudinal axis may extend along the centre of the elongate body or the instrument lumen. The centre may correspond to the centre of mass. For example, the centre may be a circle centre if the instrument lumen and/or the elongate body are circular in a cross-sectional view.

In an optional embodiment, the instrument lumen is configured to slidably receive the elongate instrument. That is, the elongate instrument is separable and detachable from the Raman spectroscopy probe. In another embodiment, the Raman spectroscopy probe includes the elongate instrument, wherein the elongate instrument is fixedly arranged in the instrument lumen such that it is not separable or detachable from the probe.

Optionally, the longitudinal axis of the elongate body coincides with the longitudinal axis of the instrument lumen.

The elongate instrument may be an endoscopic instrument configured to be inserted into and/or pulled out the instrument lumen. For example, different elongate instruments may be inserted into the instrument lumen. In particular, it is possible to push the elongate instrument out of the distal end of the elongate body, for example to use the elongate instrument at the target site while the elongate instrument remains within the instrument lumen as long as the Raman spectroscopy probe is navigated to the target site.

In this case, the instrument lumen may be defined by a wall or hollow tube, the wall are hollow tube may be configured to fluid tight seal the Raman spectroscopy probe. The inner diameter of the instrument lumen may be slightly bigger than the outer diameter of the elongate instrument such that the elongate instrument can be moved within the instrument lumen.

In another embodiment, the elongate instrument is fixed to the instrument lumen. In this case, the elongate instrument is a unitary component with the elongate body. Thus, the Raman spectroscopy probe may be used for obtaining Raman spectroscopy measurements and for utilising their capabilities of the elongate instrument. The Raman spectroscopy probe therefore has additional functionalities or capabilities.

In an optional embodiment, the elongate instrument is an (endoscopic) camera, a surgical instrument, a treatment apparatus for modifying tissue, and/or an electrosurgical treatment device for delivering electromagnetic radiation for tissue treatment.

The treatment apparatus for modifying tissue will be discussed below.

The elongate camera may be an endoscopic camera. The camera may include an image capturing device which may be configured to transform optical information of a part of the target area into electrical or digital information. For example, the image capturing device is configured to transform electromagnetic radiation, such as light, emitted from a part of the target site into an electrical or digital signal or a series of electrical or digital signals which can be processed to create a visible image of the part of the target area. The electrical or digital signal or the series of electrical or digital signals may be regarded as an electrical image or digital image of the target area. The generation of the electrical image or digital image may be executed in the region at the distal end of the electrosurgical instrument, for example by providing a CMOS sensor (array) and/or a CCD sensor (array) close to a distal end of the image capturing device. The processing of the electrical or digital signal or the series of electrical or digital signals may be executed by a display device which may also include a display for displaying the visible image of the captured part of the target area.

The image capturing device may be configured to create an optical image of the part of the target area at the region at the distal end. The image capturing device may be configured to convert the optical image of the part of the target area into an electric signal or a series of electric signals, for example digital signals, from which the image of the part of the target area can be reproduced by means of a processing apparatus, such as a computer or processor with a display screen or the display device. The conversion of the optical image created by the image capturing device into the electrical or digital image (corresponding to the electric signal or the series of electric signals) may be executed in the region at the distal end.

The image capturing device may be configured to produce an electric signal or a series of electric signals which can be transformed into a two-dimensional or three-dimensional representation of a part of the target site. This means, the camera does not necessarily transmit optical information from the distal end to the proximal end but transforms the optical information into electrical information in the region at the distal end. To this end, an optical sensor of the image capturing device is arranged near or close to the distal end of the electrosurgical instrument.

The image capturing device may include one or more lens devices and/or one or more optical sensors. The lens device may be used to project incoming light beams originating from the part of the target site onto the optical sensor (for creating an optical image of the part of the target site on the optical sensor). The lens device may include one or more optical lenses and/or an (adjustable) aperture. However, it could be that the lens device may be a pinhole. The optical sensor may be configured to convert the incoming light into electrical signals. For example, the optical sensor may be a complementary metal-oxide-semiconductor (CMOS) sensor or a charge-coupled device (CCD). The CMOS sensor may have up to 40.000 pixels. The CMOS sensor may be a quadratic or square chip having a length as well as a width down to 0.65 mm each. The lens device and/or the optical sensor may be arranged in the region at the distal end. The processing of the electrical signals generated by the optical sensor may be executed by the display device to which the image capturing device can be connected.

The image capturing device may be a camera or any other means for converting optical information into electrical information indicative of a part of the target area. The elongate camera may be configured to image visible light, ultraviolet light and/or infrared light. The elongate camera may also be configured to identify the location of markers at the target site.

The image capturing device may have a main optical axis which is defined by the respective lens device and/or the pinhole. The main optical axis optionally is parallel to the direction of a longitudinal extension of the elongate body. In this case, the image capturing device and/or the elongate camera are configured to look forwards. Alternatively or additionally, the main optical axis may be inclined to the direction of the longitudinal extension of the elongate body. For example, the image capturing device is configured to look sideward; the main optical axis may be inclined by 90° to the direction of extension, or any other angle between 0° and 90°. The lens device and/or the pinhole may be arranged on a side surface of the electrosurgical instrument or the body.

The electrical signals generated by the image capturing device may be fed to the display device. To this end, the elongate camera may include one or more cables, wires and/or other conductive components which are configured to transmit electrical signals generated by the image capturing device from the distal end of the elongate body to the proximal end. The cables, wires and/or other conductive components may extend beyond the proximal end of the elongate body and can be connected to the display device. The cables, wires and/or other conductive components may be arranged within a camera body of the elongate camera.

The display device may include a processor and/or a display. The display device may be a computer having a monitor or screen. The electrical signals generated by the image capturing device are processed by the processor and displayed by the display.

The elongate camera may be alternatively or additionally constituted by a one or more camera fibres. The camera fibres may include at least one camera illumination fibre which is configured to guide light from the proximal end to the distal end and/or at least one imaging fibre which is configured to guide light from the distal end to the proximal end. In particular, the imaging fibre extends beyond the proximal end of the elongate body and may be connected to a display device. The imaging fibres, like the collection fibres, guide the light gathered at the distal end of the elongate body to an optical sensor arranged within the display device. The optical sensor may be configured as described above. The display device may include a display for displaying a part of the target area imaged using the image capturing device.

The camera illumination fibres may also be connected to the display device which may include a camera light source. The camera light source may provide the light in the visible spectrum i.e. white light (for example between 400 nm and 700 nm) or at a wavelength range offset from the wavelength generated by the Raman light source. For example, the camera light source generates light in a narrow band, for example green light (optionally between 520nm and 560 nm) or blue light (optionally between 450 nm and 490 nm). Alternatively or additionally, the camera light source may include a white light source and one or more optical band pass filters to generate light in a narrow band, such as blue and/or green light.

The Raman light source generates light having a wavelength in the ultra-violet spectrum (for example 244 nm, 257 nm, 325 nm, 364 nm), in the visible spectrum (for example 457 nm, 473 nm, 488 nm, 514 nm, 532 nm, 633 nm, 660 nm), in the near infra-red spectrum (for example 785 nm, 830 nm, 980 nm, 1064 nm) or up to the mid infrared (IR) spectrum or far IR spectrum. A distal end of the camera illumination fibres may be configured to illuminate a part of the target area which is imaged by the image capturing device and/or the elongate camera.

The elongate camera may also include an illuminator arranged in the region at the distal end. The illuminator can include an illumination device and/or a distal end of an illumination fibre or a distal end of a plurality of camera illumination fibres. The illuminator is provided for illuminating the target area or a part of the target area, in particular that part of the target area that is captured by the image capturing device and/or the elongate camera.

The illumination device may include one or more light sources which may include one or more LEDs, for example, positioned in the region at the distal end. The illumination device, in particular the light source, may be powered by an illumination wire which is connected to the illumination device and extends to the proximal end. The illumination wire may be connected to a separate power source or may be powered by the display device.

The illuminator may include the distal end of an optical fibre(s) - the camera illumination fibre(s). The distal end of the camera illumination fibre(s) emits light that is fed into the camera illumination fibre(s) at the other end, for example at the proximal end. Thus, the illuminator forms a part of the camera fibre(s). For example, the illuminator is a lens or lens structure arranged at or fixed to the distal end of the illumination fibre or to the plurality of illumination fibres. The proximal end of the illumination fibre may be connected to a light source, such as a lamp or an LED, which can be arranged in the display device. The camera illumination fibre(s) may protrude from the proximal end of the body.

The elongate camera may include a camera body which defines an outer surface of the elongate camera. The camera body may have the same characteristics and/or features as the elongate body. In particular, all components of the elongate camera may be arranged within the camera body if the elongate camera is a separate instrument.

The outer diameter (or the maximal outer diameter) corresponds to the longest distance between any two points on the perimeter of the elongate body (or any other component to which the outer diameter refers to) in a cross sectional view.

The surgical instrument may be a needle, a scalpel or any other surgical instrument needed to treat the tissue at the target site. The surgical instrument has outer dimensions such that it can be inserted into the instrument lumen; the surgical instrument may be an endoscopic instrument.

In optional embodiment, the electrosurgical treatment device includes a coaxial transmission line configured to convey electromagnetic energy and a radiating element protruding from the transmission line and arranged to receive the electromagnet energy therefrom, the radiating element being configured to radiate the electromagnetic energy for tissue treatment.

The electrosurgical treatment device may be configured to emit radio frequency radiation and/or microwave radiation. The radio frequency radiation may be used for cutting tissue while the microwave radiation may be used to ablate, coagulate, and/or stimulate tissue at the target site. The electrosurgical treatment device optionally includes the radiating element configured to emit electromagnetic radiation and/or the transmission line to deliver the electromagnetic energy to the radiating element. The transmission line may be a coaxial cable.

The radiating element may comprise one or more conductive elements. In particular, if the radiating element is configured to emit radio frequency radiation, the radiating element includes at least two conductive elements which are arranged close to each other but isolated from one another. An electromagnetic field generated between the two conductive elements by the radio frequency electromagnetic energy supplied to the radiating element can be used to cut tissue.

The radiating element may have a dipole structure or a monopole structure; the latter may be used for emitting microwave radiation. The radiating element, preferably all conductive elements, are arranged in and/or on the body of the electrosurgical treatment device.

The radiating element may protrude from the distal end of the elongate body.

Alternatively, the radiating element may be pushed out of the instrument lumen when the electromagnetic radiation is applied to the target tissue, but may be retractable into the instrument lumen when the electromagnetic radiation is not to be applied to target tissue.

In another embodiment of the treatment device, the radiating element comprises a proximal coaxial transmission line for conveying the microwave energy and a distal needle tip mounted at a distal end of the proximal coaxial transmission line. The distal needle tip is arranged to deliver the electromagnetic energy into biological tissue.

The transmission line is connected to the radiating element for supplying electromagnetic energy from the proximal end to the radiating element. The transmission line may include any shape of conductive material in order to transmit electromagnetic energy. The transmission line may be connected to the generator at the proximal end.

In some embodiments, the proximal coaxial transmission line may comprise: an inner conductor that extends from a distal end of the flexible coaxial cable, the inner conductor being electrically connected to a centre conductor of the flexible coaxial cable; a proximal dielectric sleeve mounted around the inner conductor; and an outer conductor mounted around the proximal dielectric, wherein the distal needle tip comprises a distal dielectric sleeve mounted around the inner conductor, and wherein a distal portion of the outer conductor overlays a proximal portion of the distal dielectric sleeve.

The outer conductor may be a conductive tube, e.g. formed from nitinol, a material that exhibits longitudinal rigidity sufficient to transmit a force capable of penetrating target tissue. Preferably the conductive tube also exhibits lateral flex suitable to enable the instrument to travel through the instrument channel of a surgical scoping device. Advantageously, nitinol may provide sufficient longitudinal rigidity for piercing the duodenum wall, to enable treatment of tissue in the pancreas, whilst still providing a high degree of lateral flexibility. The distal needle tip may be substantially rigid, to facilitate insertion into biological tissue.

The inner conductor may be formed from a material with high conductivity, e.g. silver. The inner conductor may have a diameter that is less than the diameter of the centre conductor of the flexible coaxial cable. This may facilitate bending of the radiating tip portion. For example, the diameter of the inner conductor may be 0.25 mm. A preferred diameter may take into account that a dominant parameter that determines loss (and heating) along the radiating tip portion is the conductor loss, which is a function of the diameter of the inner conductor. Other relevant parameters are the dielectric constants of the distal and proximal dielectric sleeves, and the diameter and material used for the outer conductor. The dimensions of the components of the proximal coaxial transmission line may be chosen to provide it with an impedance that is identical or close to the impedance of the flexible coaxial cable (e.g. around 50 Ω).

A proximal end of the transmission line may protrude from the proximal end of the elongate body and may be connected to a generator configured to generate the electric magnetic energy.

In an optional embodiment, the transmission line is fixedly arranged in the instrument lumen such that a distal end of the transmission line coincides with the distal end of the elongate body.

In this case, the radiating element of the treatment device protrudes from the distal end of the elongate body. The transmission line is arranged within in the elongate body and can be isolated from the surroundings by the elongate body.

For example, in case of a coaxial cable as the transmission line, the outer insulation sheath of the coaxial cable may be the wall or tube defining the instrument lumen. The transmission line may be fixed to the wall or tube defining the instrument lumen or, in the absence of the wall of tube, the transmission line may be fixed to the ring of fibres and or the filler material arranged within the elongate body. The fixation may be achieved by using an adhesive.

In an optional embodiment, the transmission line includes a hollow inner conductor wherein preferably, the radiating element is retractable into a distal end of the hollow inner conductor.

The transmission line may be a coaxial cable having a hollow inner conductor, an outer conductor and a dielectric material insulating the inner conductor from the outer conductor. The radiating element may be placed within the hollow inner conductor when the Raman spectroscopy probe is navigated to the target area. The radiating element is pushed out of the hollow inner conductor once the distal end of the Raman spectroscopy probe is arranged at the target site in order to emit the electromagnetic radiation.

In optional embodiment, the Raman spectroscopy probe further comprise a cap device mounted to the distal end of the elongate body and comprising an optical structure optically coupled to the at least one illumination fibre and/or the at least one collection fibre wherein preferably the optical structure includes at least one lens.

The optical structure of the cap device may be a window through which the light from the illumination fibre can reach to target area and/or the Raman scattered light can couple into the collection fibre. The at least one illumination fibre and/or the at least one collection fibre may be fixed to the cap device such that the fibres cannot be moved relative to the cap device. The cap device may additionally configured to seal the distal end of the elongate body. The cap device may be dome-shaped.

Alternatively or additionally, the optical structure include one or more lenses or lens structures which focus the light emitted from the illumination fibres onto the target area and/or focus the Raman scattered light into the collection fibres. The optical structure may further include optical filters.

In an optional embodiment, the cap device includes an aperture which is aligned with the instrument lumen. In an optional embodiment, the cap device retains a distal end of the transmission line within the elongate body. Also, the cap device may include a centraliser.

The aperture is provided such that the elongate instrument may protrude from the distal end of the elongate body. Furthermore, the cap device may additionally cover parts of the instrument lumen, in particular a ring contacting the circumference of the instrument lumen in a cross-sectional view. For example, the radiating element protrudes through the aperture of the cap device. An outer diameter of the radiating element is smaller than an outer diameter of the transmission line. In this case, the inner diameter of the aperture corresponds to the outer diameter of the radiating element and, thus, is smaller than the outer diameter of the transmission line. Thus, the cap also covers that part of the transmission line which radially extends beyond the diameter of the radiating element.

The centraliser is an electrical component for securing the radiating element to the flexible transmission line (coaxial cable). The centraliser may include a collar mounted over a junction therebetween. The collar may be electrically conductive, e.g. formed from brass. It may electrically connect the outer conductor of the proximal transmission line to the outer conductor of the flexible coaxial cable (transmission line).

Thus, the cap device may have a double function of including optical components for the at least one collection fibre and/or the at least one illumination fibre and of securing the outer connector of the radiating element to the outer conductor of the transmission line.

In an optional embodiment, the Raman spectroscopy probe further comprises at least one imaging fibre within the elongate body for guiding light from the distal end to the proximal end, the at least one imaging fibre being arranged outside the instrument lumen.

The imaging fibre may be part of the elongate camera as described above. The camera fibre includes at least one imaging fibre and/or at least one camera illumination fibre. The imaging fibre and/or the camera illumination fibre may be mixed with the illumination fibre and collection fibre within the space defined by the outer surface the instrument lumen and the inner surface of the elongate body. For example, the at least one camera fibre, in particular at least one imaging fibre, is arranged in the second lumen. The arrangement and number of these different types of fibres may be chosen to achieve optimum imaging and Raman spectroscopy capabilities.

In an embodiment, a plurality of camera fibres are provided which may be arranged in a ring in a cross-sectional view. Each camera fibre may extend at least from the proximal end through the elongate body to the distal end. A plurality of rings of camera fibres may be provided. The ring of camera fibres may surround the illumination fibres and/or collection fibres.

The camera illumination fibres may form a ring of fibres which can be positioned radially outside a radially inside of the ring of imaging fibres.

In an optional embodiment, the Raman spectroscopy probe further comprises a third lumen extending between the distal end and the proximal end, wherein preferably the at least one camera fibre, in particular at least one imaging fibre, is arranged in the third lumen.

The third lumen may be delimited from the instrument lumen and/or the second lumen by an additional wall (for example the middle wall) arranged within the elongate body and extending from the distal end to the proximal end. In this embodiment, the illumination fibres and collection fibres are separated from the camera fibres, in particular the imaging fibres, by the wall defining the third lumen.

For example, the second lumen and/or the third lumen may be hollow cylinders; in other words, the second lumen and/or the third lumen are rings in a cross-sectional view of the elongate body. The wall separating the third lumen from the second lumen may be in this case a hollow tube. The third lumen and the second lumen may be coaxially arranged.

In an optional embodiment, in a cross-sectional view of the elongate body, a plurality of camera fibres are provided which distributed around a circumference of the instrument lumen and, when preferably the plurality of camera fibres are distributed around a majority or entirety of the circumference of the instrument lumen.

For example, the plurality of imaging fibres and/or camera illumination fibres may form a ring or a section of a ring which is coaxially arranged with the instrument lumen. In an optional embodiment, this ring or a section of a ring of imaging fibres and/or camera illumination fibres may be arranged in the third lumen as described above.

The imaging fibres may be arranged in a single ring. Similarly, the camera illumination fibres may also be arranged in a single ring. Furthermore, the imaging fibres and camera illumination fibres may be arranged in a single ring. Basically the same remarks regarding the arrangement of the illumination fibre and collection fibres may be equally applied to the imaging fibres and camera immigration fibres, respectively.

In an optional embodiment, each imaging fibre includes a lens structure at its distal end, the lens structure being configured to couple light into the imaging fibres.

The lens structure may be manufactured by grinding the distal end of the imaging fibres. Alternatively or additionally, the lens structure may be attached to the distal end of the imaging fibres.

In an optional embodiment, the imaging fibre and/or the camera illumination fibre are coupled to the optical structure of the cap device.

The optical structure may include additional lenses and/or other optical components for the imaging fibres and/or the camera illumination fibres. In other words, the lens structure is optically coupled to the imaging fibres and/or the camera illumination fibres. The lens structure for the imaging fibres and/or camera illumination fibres may be an optically transparent window. In this case, the lenses and/or other optical components may be attached to the distal end of the imaging fibre and/or the camera illumination fibre. The window may be made from fused silica or Magnesium fluoride (MgF₂)

In an optional embodiment, the analysis device further includes an illumination source for generating non-monochromatic light and an optical illumination component, wherein the optical illumination component is connected to the at least one Raman fibre, the Raman light source and the illumination source, and wherein the optical illumination component is configured to selectively or permanently couple the monochromatic light from the Raman light source and/or the non-monochromatic light from the illumination source into the at least one Raman fibre.

The illumination source may correspond to the camera light source, however it is not arranged in the display device, but in the analysis device. This may be beneficial if the elongate instrument is an elongate camera which does not provide illumination of the target site; the illumination of the target site is achieved by using the illumination source and the at least one Raman fibre. For example, the elongate camera does not include an illuminator or solely includes imaging fibres connected to the optical sensor of the display device. The illumination source is optionally coupled to the at least one illumination fibre. The illumination source may have the same characteristics and/or features as the camera light source.

The optical illumination component may be an optical switch in one embodiment, for example a single-pole-double-pole switch. The optical switch may be configured to selectively switch the light source that is inputted into the at least one Raman fibre. For example, when performing Raman spectroscopy measurements, the optical switch couples the light of the Raman light source into the at least one Raman (illumination) fibre. When imaging the target site using the elongate camera, the optical switch couples the light from the illumination source into the at least one Raman (illumination) fibre. In an arrangement where the same fibres are used and the sources are switched, the measurements should be made fast enough to prevent the vision side of the system being impaired, i.e. the measurement may need to be made in a 10ms time slot every second.

It may also be possible that the optical switch simultaneously or concurrently couples both the light from the Raman light source and the light from the illumination source into the at least one Raman (illumination) fibre - for example when imaging and Raman spectroscopy measurements are simultaneously performed.

In another embodiment, the optical illumination component may be an optical coupler which permanently couples the lights from both the Raman light source and the illumination source into the at least one Raman (illumination) fibre. The optical coupler may be a Y-branch coupler. Depending on the function to be performed, the Raman light source and/or the illumination source are selectively switched on or off.

In an optional embodiment, the analysis device further includes an optical detection component, wherein the optical detection component is connected to the at least one Raman fibre, the Raman light source and the spectrometer, and wherein the detection component is configured to route the monochromatic light of the Raman light source into the at least one Raman fibre and the light from the at least on Raman fibre to the spectrometer.

The optical detection component may include a time division switch and/or a multiplexer. The optical detection component may be configured to route light depending on the wavelength, polarization, and/or phase. This may help to reduce/filter out Rayleigh scattered light from the Raman scattered light. However, the optical detection component may include a coupler and, optionally, some optical filters.

In this embodiment, only one type of Raman fibres is provided, for example only collection fibres. The same Raman fibres are used for guiding light from the proximal end to the distal end as well as from the distal end to the proximal end.

The optical detection component couples light from the Raman light source into the Raman fibre. In addition, the optical detection component directs Raman scattered light to the spectrometer. This functionality may be selectively performed, for example temporally separated or depending on the respective wavelength. The optical detection component may help to reduce the number of Raman fibres or increase the number of fibres which act as collection fibres, since each fibre acts as a collection fibres.

Furthermore, the invention refers to a combined Raman spectroscopy probe and a direct vision camera, e.g. a miniature CMOS camera with suitable white light or narrow band imaging. This combined Raman spectroscopy and direct vision that takes place in the visible region of the EM spectrum may also form a stand-alone device as well as a device that is integrated with a therapeutic energy source.

To this end, the elongate camera described above may be permanently fixed to the instrument lumen. The elongate camera may include the image capturing device as described above. The image capturing device may include the CMOS sensor. As described above, the illumination for imaging may be provided by the camera illumination fibres and/or by using the illumination fibres for illuminating the target site (as above).

For example, a ring of camera illumination fibres surrounds the elongate camera and, thus, the instrument lumen. The ring of camera illumination fibres may be surrounded by a ring of illumination fibres which in turn may be surrounded by a ring of collection fibres. However, other arrangements of the different types of fibres are possible as discussed above.

In another embodiment, a ring of illumination fibres surrounds the elongate camera and, thus, the instrument lumen. The ring of illumination fibres may be surrounded by a ring of collection fibres. However, other arrangements of the different types of fibres are possible as discussed above. In this embodiment, the illumination fibres may be used for providing illumination for both imaging and Raman measurements (see above).

The Raman spectroscopy apparatus according to the current invention further comprises a treatment light source configured to generate light having an intensity configured to modify tissue. At least one treatment fibre may be provided within the elongate body. Optionally the at least one treatment fibre is arranged outside the instrument lumen. A proximal end of the least one treatment fibre may be coupled to the treatment light source. The least one treatment fibre may be configured to guide the light from the treatment light source to the distal end.

The treatment light source may include a laser, in particular a semiconductor laser. The treatment light source may generate light having a wavelength in the visible spectrum, for example between 380 nm and 700 nm. The intensity of the (laser) light generated by the treatment light source may be sufficient to modify, in particular cut coagulate and/or ablate, tissue within the human body. In particular, the intensity of the light generated by the treatment light source may be sufficient to cut soft tissue such as muscle tissue or cancerous tissue within organ. The intensity of the light generated by the treatment light source may only be high enough to modify tissue in a focal point where the laser light is focused on to a small volume.

The light generated by the treatment light source may be continuous wave (CW) or pulsed. The power in the CW mode may be from around 1W to 100W. In the pulsed mode, the power could be 10W to 1000W pulsed with a 10% duty cycle.

The treatment light source may be configured to generate light of a single wavelength or can be configured to generate light of multiple wavelengths. To this end, the treatment light source may include one or more lasers, in particular semiconductor lasers. The output of the treatment light source is coupled into the treatment fibre or the plurality of treatment fibres. To this end, one or more lenses, a lens system (e.g. collimating lens arrangement) and/or other optical components may be provided for coupling the laser light generated by the treatment light source into the treatment fibre.

The treatment fibre may be configured to propagate the light generated by treatment light source with little to no absorption. For example, the absorption of laser light by the treatment fibre may be below 5%, below 3% or below 1%. The absorption may be below 0.1 dB/m. The treatment fibre may be an optical fibre having an outer diameter of 0.2 mm to 2 mm. The treatment fibre may have a larger diameter compared to the Raman fibre or camera fibre. The length of the treatment fibre, the Raman fibre and/or the camera fibre may be up to 2.5 m.

The Raman spectroscopy probe may include at least one treatment fibre for guiding light between the proximal end and the distal end, wherein the at least one treatment fibre is arranged outside of the instrument lumen.

The at least one treatment fibre is arranged outside of the instrument lumen but within the elongate body of the Raman spectroscopy apparatus. The treatment fibre or the plurality of treatment fibres may be arranged in configurations similar to the ones of the Raman fibres and/or camera fibres. Thus, the same definitions, features and/or characteristics as discussed above apply to the treatment fibres. The treatment fibre(s) may be arranged between the Raman fibres and/or illumination fibres. For example, the at least one treatment fibre may be placed into the above described configurations of Raman fibres and/or camera fibres by replacing one or more of the Raman fibres and/or camera fibres by one or more treatment fibres.

A distal end of the treatment fibre may be provided with a lens or lens system for focusing the light emitted by the treatment fibre onto the tissue in order to enhance the intensity in the focal point of the focused light. The lens or lens system may be alternatively included in the cap device. Thus, the lens or lens system for focusing the light emitted by the treatment fibre may be part of the optical structure of the cap device.

The treatment light source and the treatment fibre may be provided for modifying, in particular cutting, tissue at the target site. For example, cancerous tissue may be identified using Raman spectroscopy while the cancerous tissue is cut using light generated the treatment light source and/or the surgical procedure is monitored using the camera and the camera fibres. Thus, the Raman spectroscopy apparatus of this embodiment is a surgical instrument for simultaneously analysing, treating and monitoring a target site. What is more, the optical elements arranged at the distal end of the Raman fibre, the camera fibre and the treatment fibre can be specifically adapted to the desired characteristics to be achieved with each fibre.

According to the invention, the Raman spectroscopy apparatus further comprises a treatment light source configured to generate light having an intensity configured to modify tissue and an optical coupler component. An output of the optical coupler component may be coupled to the at least one Raman fibre and/or the least one camera fibre. Inputs of the optical coupler component may be coupled to two of: the Raman light source, the illumination source, and the treatment light source. The optical coupler component is configured to selectively or permanently couple the light from the Raman light source and the light from the treatment light source into the at least one Raman fibre or light from the illumination source and the light from the treatment light source into the at least one camera fibre.

In this embodiment of the Raman spectroscopy apparatus, the light emitted by the treatment light source is not guided to the target site via a special (additional) treatment fibre. Instead, the Raman fibres and/or the camera fibres may be used for guiding light generated by the treatment light source to the target site. To this end, the light generated by the treatment light source may be coupled into the Raman fibres and/or camera fibres by the optical coupler component. For example, if the Raman light source and/or the illumination source is switched off, the light generated by the treatment light source may be coupled into the Raman fibres and/or the camera fibres. It is also possible that the light generated by the treatment light source is coupled into the Raman fibres and/or the camera fibres while light generated by the Raman light source and/or the camera light source is coupled into the respective fibres. This allows to reduce the number of optical fibres and, thus, to reduce the outer diameter of the elongate body.

The optical coupler component may be configured similar to the illumination component described above. Thus, the characteristics and definitions described above equally apply to the optical coupler component.

In one embodiment, the optical coupler component is arranged to selectively or permanently couple the light generated by the Raman light source and a treatment light source into the Raman fibre, in particular the illumination fibre. Thus, the two inputs of the optical coupler component are coupled to the Raman light source and treatment light source, respectively. The output of the optical coupler component is coupled to the Raman fibre. In another embodiment, the optical coupler component is arranged to selectively or permanently couple light generated by the treatment light source and the camera light source into the camera fibre, in particular the camera illumination fibre. Thus, the two inputs of the optical coupler component are coupled to the camera light source and treatment light source, respectively. The output of the optical coupler component is coupled to the camera fibre.

In an optional embodiment, the Raman light source and/or the illumination source are configured to generate light having varying intensity, wherein preferably the Raman spectroscopy apparatus further comprises a controller for controlling the intensity of the light generated by the Raman light source and/or the illumination source.

In this embodiment, the treatment light source is provided by the Raman light source and/or the illumination source. To this end, the Raman light source and/or the illumination source are configured to generate light having intensities as described in conjunction with the treatment light source. For example, the intensity of the light generated by the Raman light source and/or the illumination source is the maximum intensity when modifying tissue. If the treatment (cutting) capabilities are not needed, the Raman light source and/or the illumination source are set to generate light having significantly lower intensities.

A controller is provided for controlling the illumination source and/or the Raman light source. In particular, the controller is configured to adjust the intensity of the light generated by the illumination source and/or the Raman light source.

According to this embodiment, the provision of a separate treatment light source is not necessary. The light for modifying the tissue has the same wavelength as the light generated by the Raman light source and/or the illumination source.

The application also refers to a treatment apparatus for modifying tissue, which comprises a treatment light source configured to generate light having an intensity configured to modify tissue and/or at least one treatment fibre having a distal end and a proximal end. The proximal end of the least one treatment fibre may be coupled to the treatment light source. The least one treatment fibre may be configured to guide the light generated by the treatment light source from the proximal end to the distal end.

The treatment apparatus may be a separate apparatus, in particular separate from the Raman spectroscopy apparatus described above. The treatment fibre of the treatment apparatus may be configured to be inserted into the instrument lumen of the elongate body of the Raman spectroscopy apparatus. Alternatively, the treatment fibre of the treatment apparatus may be permanently fixed to the instrument lumen of the elongate body of the Raman spectroscopy apparatus.

The treatment apparatus may be used for treatment throughout the bronchial tree, the pancreas and/or the brain. The treatment light source and/or the treatment fibre may have to same characteristics and features as described above.

An elongate assembly comprises a coaxial feed cable and a radiating tip. The coaxial feed cable is configured to convey electromagnetic energy and includes an inner conductor, an outer conductor, and a dielectric material separating the inner conductor and the outer conductor. The radiating tip is disposed at a distal end of the coaxial feed cable to receive the electromagnetic energy therefrom. The radiating tip is configured to radiate the electromagnetic energy for tissue treatment. The inner conductor and the radiating tip include a passageway configured to receive an elongate instrument. The radiating tip comprises an elongate conductor electrically connected to the inner conductor and extending in a longitudinal direction to form an electromagnetic radiator, a first tuning element electrically connected to the elongate conductor, and a dielectric body disposed around the elongate conductor and the first tuning element. An electromagnetic field emitted by the electromagnetic radiator is shaped around the dielectric body.

In an optional embodiment, the elongate instrument is a surgical instrument, an endoscopic, a treatment apparatus for modifying tissue and/or elongate camera or an elongate Raman spectroscopy instrument comprising at least one Raman fibre extending between a distal end of the Raman spectroscopy instrument and a proximal end of the Raman spectroscopy instrument, the at least one Raman fibre optionally including illumination fibre for guiding light from a proximal end to a distal end and/or at least one collection fibre for guiding light from the distal end to the proximal end.

The surgical instrument, the treatment apparatus for modifying tissue, and/or the elongate camera may have the characteristics and configurations as described above.

The Raman fibre, the illumination fibre and/or collection fibre of the elongate Raman spectroscopy instrument may have the characteristics and configurations as described above. The Raman spectroscopy instrument may be the Raman spectroscopy probe as described above. The Raman spectroscopy instrument may be used instead of the Raman spectroscopy probe in the Raman spectroscopy apparatus. Thus, the Raman spectroscopy apparatus includes the Raman spectroscopy instrument and the analysis device.

The illumination fibre and collection fibre of the Raman spectroscopy instrument may be arranged in a tightly packed bundle encouraged by an elongate body as described above. In particular, the Raman spectroscopy instrument may not include the instrument lumen.

The elongate assembly may be an instrument for ablating, coagulating, cutting and/or stimulating tissue by the emission of electromagnetic radiation, in particular radio frequency radiation and/or microwave radiation. A further functionality may be provided by inserting the elongate instrument through the passageway. For example, the elongate camera and/or the Raman spectroscopy instrument may be used for imaging/analysing the tissue which is treated by the radiation emitted by the radiating tip.

The elongate instrument may be slidably inserted into the passageway such that it can be pushed or pulled out of the distal end of the radiating tip for imaging/analysing the tissue to be treated with the electromagnetic radiation. Thus, it is possible to simultaneously insert the device for ablating/cutting tissue with an imaging or analysis device. This simplifies the insertion process as various functionalities can be simultaneously inserted.

In an optional embodiment, the elongate further comprises a second tuning element electrically connected to the elongate conductor in a distal region of the radiating element, wherein the first tuning element is positioned in a proximal region of the radiating tip, wherein the dielectric body is disposed around the second tuning element, and wherein the first tuning element and the second tuning element are spaced apart in the longitudinal direction, whereby the microwave field emitted by the microwave radiator is shaped around the dielectric body.

The elongate assembly may operate to ablate target tissue in the body. The device is particularly suited to the ablation of tissue in the lungs, however it may be used to ablate tissue in other organs (e.g. the uterus or the GI tract). In order to efficiently ablate target tissue, the radiating tip should be located as close as possible (and in many cases inside) the target tissue. In order to reach the target tissue (e.g. in the lungs), the device may need to be guided through passageways (e.g. airways) and around obstacles. This means that the instrument will ideally be as flexible as possible and have a small cross section. Particularly, the device should be very flexible near its tip, where it may need to be steered along narrow passageways such as bronchioles which can be narrow and winding.

The coaxial feed cable may be a conventional low loss coaxial cable that is connectable at one end to an electrosurgical generator. In particular, the inner conductor may be an elongate conductor extending along a longitudinal axis of the coaxial feed cable. The dielectric material may be disposed around the inner conductor, e.g. the first dielectric material may have a channel through which the inner conductor extends. The outer conductor may be a sleeve made of conductive material that is disposed on the surface of the dielectric material. The coaxial feed cable may further include an outer protective sheath for insulating and protecting the cable. In some examples, the protective sheath may be made of or coated with a nonstick material to prevent tissue from sticking to the cable. The radiating tip is located at the distal end of the coaxial feed cable, and serves to deliver EM energy conveyed along the coaxial feed cable into target tissue. The radiating tip may be permanently attached to the coaxial feed cable, or it may be removably attached to the coaxial feed cable. For example, a connector may be provided at the distal end of the coaxial feed cable, which is arranged to receive the radiating tip and form the required electrical connections.

The radiating tip may be generally cylindrical. The dielectric body may be attached to a distal end of the coaxial feed cable. In some examples, the dielectric body may comprise a protruding portion of the dielectric material of the coaxial feed cable that extends beyond the distal end of the coaxial feed cable. This may simplify construction of the radiating tip, and avoid reflections of EM energy at the boundary between the radiating tip and the coaxial feed cable. In other examples, a second dielectric material, different from the dielectric material of the coaxial feed cable may be used to form the dielectric body. The second dielectric material may be selected to improve impedance matching with target tissue in order to improve the efficiency with which the microwave energy is delivered into target tissue. The radiating tip may also include multiple different pieces of dielectric material, which are selected and arranged to shape the radiation profile in a desired manner.

The elongate conductor is electrically connected to the inner conductor of the coaxial feed cable and extends within the dielectric body so that it acts as a microwave radiator. In other words, microwave energy conveyed to the radiating tip from the coaxial feed cable may be radiated from the elongate conductor. The outer conductor may terminate at the distal end of the coaxial feed cable, such that the elongate conductor extends beyond a distal end of the outer conductor. In this manner, the radiating tip may act as a microwave monopole antenna. Thus, microwave energy conveyed to the radiating tip may be radiated from the elongate conductor into surrounding target tissue. The elongate conductor may, for example, extend within a channel in the dielectric body. The elongate conductor may be any suitable conductor having an elongate shape. For example, the elongate conductor may be a wire, rod or strip of conductive material that extends within the dielectric body.

The first tuning element may be called proximal tuning element and can be a piece of conductive material (e.g. metal) that is located near a proximal end of the radiating tip. The second tuning element may be called distal tuning element and can be a piece of conductive material (e.g. metal) that is located near a distal end of the radiating tip. Thus, the distal tuning element may be further away from the distal end of the coaxial feed cable than the proximal tuning element. The proximal and distal tuning elements are both electrically connected to the elongate conductor. For example, the proximal and distal tuning elements may each be disposed on or around the elongate conductor. The proximal and distal tuning elements may be electrically connected to the elongate conductor by any suitable means. For example, the proximal and distal tuning elements may be welded or soldered to the elongate conductor. In another example, the proximal and distal tuning elements may be connected to the elongate conductor using a conductive adhesive (e.g. conductive epoxy). Alternatively, one or both of the proximal and distal tuning elements may be integrally formed with the elongate conductor (e.g. they may be manufactured together as a single piece). The proximal and distal tuning elements are spaced apart in a longitudinal direction by a length of the elongate conductor. In other words, a section of the elongate conductor is disposed between the proximal and distal electrodes. The proximal and distal tuning elements may be covered by a portion of the dielectric body, so that they are isolated/protected from the environment.

The inventors have found that a radiating tip having a configuration as described above may reduce an impedance mismatch between the radiating tip and surrounding target tissue. This may reduce the amount of microwave energy that is reflected back down the coaxial feed cable at the radiating tip (which occurs due to impedance mismatch between the radiating tip and the target tissue). As a result, the efficiency with which microwave energy can be delivered into target tissue may be improved. This may enable the amount of energy that needs to be conveyed down the coaxial feed cable to ablate target tissue to be reduced. This may in turn reduce heating effects due to transmission of microwave energy along the coaxial feed cable, such that the electrosurgical instrument may be used for longer periods of time.

The inventors have also found that the proximal and distal tuning elements may result in a more desirable radiation profile of the radiating tip. In particular, the tuning elements may shape the radiation profile such that it is concentrated around the radiating tip, and reduce a tail of the radiation profile that extends back along the coaxial feed cable. In this manner, microwave energy conveyed to the radiating tip may be emitted from the radiating tip and ablate surrounding target tissue in a welldefined volume around the radiating tip. The ablation volume (i.e. a volume of tissue that is ablated by the radiated microwave energy) may be approximately spherical. The shape, size and location of the tuning elements may be selected to obtain a desired microwave radiation profile.

The proximal tuning element and the distal tuning element may be disposed symmetrically with respect to the longitudinal direction. For example, the proximal tuning element and the distal tuning element may be cylindrical, e.g. having a central axis that is collinear with a longitudinal axis of the elongate conductor. The longitudinal axis of the elongate conductor is an axis along the length of the elongate conductor. For example, the proximal tuning element may be a cylindrical piece of conductive material disposed around, and coaxial with, the elongate conductor. This may improve the axial symmetry of the radiation profile of the radiating tip. In some embodiments, the proximal tuning element may be spaced from the distal end of the coaxial feed cable in the longitudinal direction. For example, the dielectric body may include a spacer which is positioned between the distal end of the coaxial feed cable and the proximal tuning element. The inventors have found that spacing the proximal tuning element from the distal end of the coaxial feed cable may introduce a phase shift into the instrument. The phase shift may improve impedance matching between the radiating tip and target tissue, so that efficiency of microwave energy into target tissue may be improved. The phase shift may depend on the distance between the distal end of the coaxial feed cable and a proximal end of the proximal tuning element.

The passageway extends to the distal end of the elongate assembly and is open at the distal end of the elongate assembly. Thus, the elongate instrument can be pushed out of the distal end of the passageway. The inner conductor of the radiating tip may be hollow to provide the passageway. The inner conductor of the coaxial cable and the inner conductor of the radiating tip may be aligned to form the passageway.

The passageway may have an inner diameter in the range of 1.5 mm to 2.3 mm, preferably 1.8 mm to 2.0 mm. The outer diameter of the elongate instrument is slightly smaller than the inner diameter of the passageway.

The application also refers to a use of a treatment device having a radiating element or radiating tip configured to emit electromagnetic radiation, in particular radiofrequency or microwave radiation. The treatment device is used in that the radiating element or radiating tip is arranged close to or in contact with a cancerous node, preferably in a lung tissue, and then electromagnetic radiation is emitted by the radiating element or the radiating tip in such a way that the tissue of the cancerous node is not permanently damaged.

The treatment device may be an endoscopic instrument having an elongate body with a proximal end and distal end. The radiating element may be arranged at or close to the distal end. The radiating element may be connected to a generator via a feed structure arranged within the elongate body. Thus, the distal end is navigated to the cancerous node either by itself or by using a catheter in which the treatment device is inserted to.

The electromagnetic radiation may have such an energy level and/or the time of application of the electromagnetic radiation may be so short that the tissue in the cancerous node is not permanently damaged. For example, the tissue in the cancerous node is heated to a temperature at which the tissue is not damaged. The temperature to which the cancerous node is heated is below the temperature at which degeneration of the molecules within the cancerous tissue occurs. The temperature of the tissue may be monitored to ensure that the tissue is not permanently damaged. With other words, after while (for example after the treated tissues returns to the body temperature), it cannot be determined whether the node was subjected to the electromagnetic radiation or not.

The inventors found that, although the cancerous tissue was not physiologically modified by the electromagnetic radiation, changes in the cancerous node can be observed; for example, a healing process of the cancerous node is started. It is thought that the application of the electromagnetic radiation stimulates a response by the immune system. What is more, it was found that cancerous nodes in the vicinity of the treated cancerous node but not treated with electromagnetic radiation themselves also experience changes resulting in a reduction of the cancerous tissue. This effect is considered to be induced by the immune response triggered by the application of electromagnetic radiation.

The treatment device can be the Raman spectroscopy probe including the electrosurgical treatment device or the elongate assembly as described above. The cancerous tissue may be detected by using Raman spectroscopy facilitated by the Raman spectroscopy probe while electromagnetic energy is emitted by the electrosurgical treatment device arranged within the instrument lumen. The treatment device may be configured as described in WO 2020/011547 or WO 2020/221749.

In this specification "microwave" may be used broadly to indicate a frequency range of 400 MHz to 100 GHz, but preferably the range 1 GHz to 60 GHz. Preferred spot frequencies for microwave EM energy include: 915 MHz, 2.45 GHz, 3.3 GHz, 5.8 GHz, 10 GHz, 14.5 GHz and 24 GHz. 5.8 GHz may be preferred.

The term "electrosurgical" is used in relation an instrument, apparatus or tool which is used during surgery and which utilises microwave and/or radiofrequency electromagnetic (EM) energy.

### BRIEF DESCRIPTION OF FIGURES

Embodiments of the invention will be discussed in conjunction with the accompanying drawings. Therein,
- Fig. 1: shows a schematic perspective view of a Raman spectroscopy apparatus including a Raman spectroscopy probe;
- Fig. 2: shows a cross-sectional view of the Raman spectroscopy probe according to Fig. 1;
- Fig. 3: shows a another cross-sectional view of the Raman spectroscopy probe according to Fig. 1;
- Fig. 4: shows a cross-sectional view of a Raman spectroscopy probe according to a second embodiment;
- Fig. 5: shows a cross-sectional view of a Raman spectroscopy probe according to a third embodiment;
- Fig. 6: shows a another cross-sectional view of the Raman spectroscopy probe according to Fig. 5;
- Fig. 7: shows a schematic perspective view of a further embodiment of a Raman spectroscopy apparatus including of a Raman spectroscopy probe according to a fourth embodiment;
- Fig. 8: shows a cross-sectional view of the Raman spectroscopy probe according to Fig. 7;
- Fig. 9: shows another cross-sectional view of a Raman spectroscopy probe according to Fig. 7;
- Fig. 10: shows a cross-sectional view of a Raman spectroscopy probe according to a fifth embodiment;
- Fig. 11: shows a schematic perspective view of another embodiment of a Raman spectroscopy apparatus including a Raman spectroscopy probe;
- Fig. 12: shows a cross-sectional view of the Raman spectroscopy probe according to Fig. 11;
- Fig. 13: shows a schematic perspective view of a further embodiment of a Raman spectroscopy apparatus including a Raman spectroscopy probe according to a sixth embodiment;
- Fig. 14: shows a cross-sectional view of the Raman spectroscopy probe according to Fig. 13;
- Fig. 15: shows another cross-sectional view of a Raman spectroscopy probe according to Fig. 13;
- Fig. 16: shows a schematic perspective view of a further embodiment of a Raman spectroscopy apparatus including a Raman spectroscopy probe according to a seventh embodiment;
- Fig. 17: shows a schematic perspective view of a further embodiment of a Raman spectroscopy apparatus including a Raman spectroscopy probe according to an eighth embodiment;
- Fig. 18: shows a cross-sectional view of the Raman spectroscopy probe according to Fig. 17;
- Fig. 19: shows another cross-sectional view of a Raman spectroscopy probe according to Fig. 17;
- Fig. 20: shows a schematic perspective view of a further embodiment of a Raman spectroscopy apparatus including a Raman spectroscopy probe according to a ninth embodiment;
- Fig. 21: shows a schematic perspective view of a treatment apparatus; and
- Fig. 22: shows a schematic perspective view of a further embodiment of an assembly.

### DESCRIPTION OF SOME EMBODIMENTS OF THE INVENTION

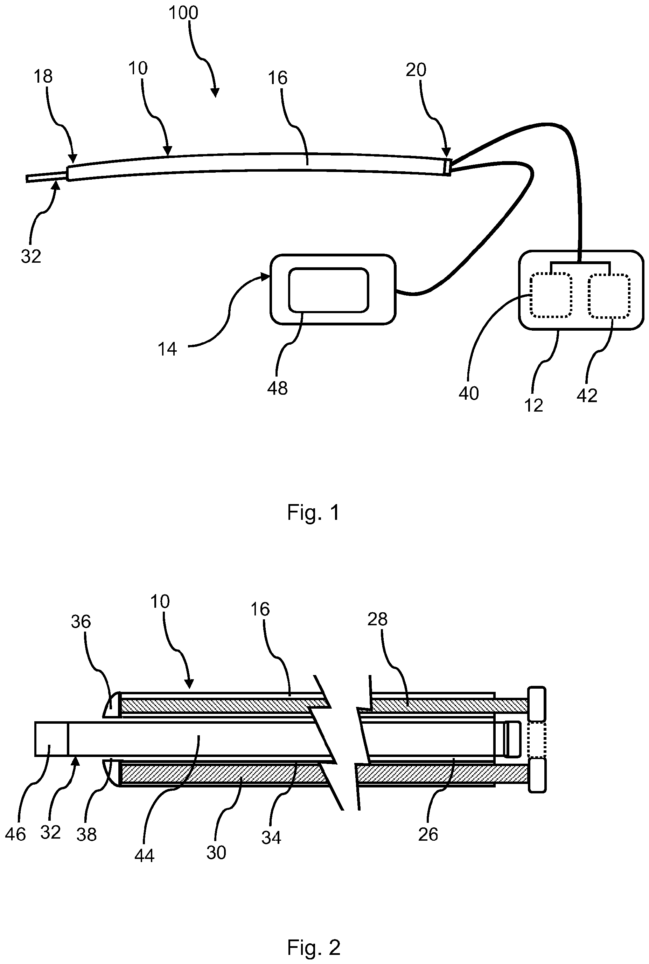

Fig. 1 shows a Raman spectroscopy apparatus 100 which includes a Raman spectroscopy probe 10, an analysis device 12, and/or a display device 14. The Raman spectroscopy probe 10 includes an elongate body 16 which extends from a distal end 18 to a proximal end 20. The Raman spectroscopy probe 10, and in particular the elongate body 16, has an elongated structure and is configured to be inserted into a cavity of a body. The Raman spectroscopy probe 10 may be an endoscopic device and can be bended or flexed in order to navigate the distal end 18 through the cavity of the body to a target site or target area. The target site may be a region within the cavity of the body (for example the lung) which is intended to be analysed, monitored and/or treated.

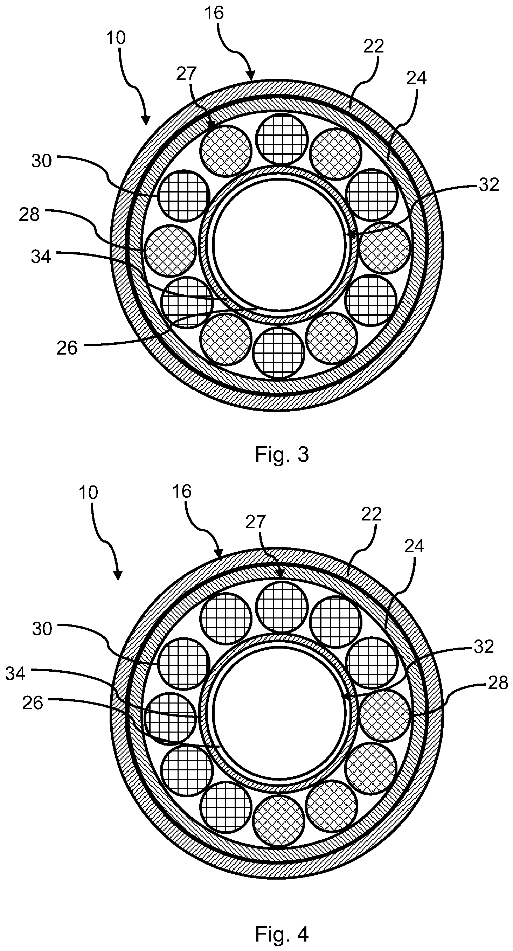

The elongate body 16 covers the Raman spectroscopy probe 10 from the distal end 18 to the proximal end 20. In particular, the elongate body 16 insulates the inside of the Raman spectroscopy probe 10 from its surrounding, for example from fluids and the like. As better visible in Fig. 3, the elongate body 16 includes an outer sheath 22 and a strain relief layer 24. The outer sheath 22 may be made from a lubricous polymer in order to reduce the friction when inserting the Raman spectroscopy probe 10 into the cavity of the body or scoping device such as a catheter. The strain relief layer 24 may include a braid, a coil, and/or tube made of a polymer or metal wire which each or in combination limit the angle of flexion of the Raman spectroscopy probe 10 such that components arranged within the elongate body 16 (to be described later) are not damaged when flexed or bent.

The Raman spectroscopy probe 10 further includes an instrument lumen 26 and a Raman fibre 27 which may include at least one illumination fibre 28 and/or at least one collection fibre 30. The instrument lumen 26 extends from the distal end 18 to the proximal end 20 and is arranged within the elongate body 16. The instrument lumen 26 may be considered a passageway in which an elongate instrument 32 can be slidably arranged. In other words, the elongate instrument 32 can be inserted into or pulled out of the instrument lumen 26. An inner diameter of the instrument lumen 26 is slightly greater than an outer diameter of the elongate instrument 32.

The instrument lumen 26 may be delimited by a hollow tube 34 which extends from the distal end 18 to the proximal end 20 and acts as a wall to seal the Raman spectroscopy probe 10, for example from fluids entering the space between the tube 34 and the elongate body 16. The hollow tube 34 may be made from a plastic material, for example from the same material as the sheath 22. The lubricous polymer from which the hollow tube 34 may be made can contribute to the reduction of the friction between the elongate instrument 32 and the hollow tube 34.

As better visible in Fig. 3, the instrument lumen 26 and, thus, the hollow tube 34 are coaxially arranged with the elongate body 16. Optionally, the instrument lumen 26 and/or the hollow tube 34 are centrally arranged within the elongate body 16. However, the invention is not limited to this embodiment. The instrument lumen 26 is arranged inside the elongate body 16 while the at least one illumination fibre 28 and at least one collection fibre 30 are arranged outside of the instrument lumen 26, in particular between the instrument lumen 26 and the elongate body 16.

In the embodiment shown in the figures, a plurality of illumination fibres 28 and collection fibres 30 (completely) surrounds the instrument lumen 26. As visible in Figs. 3 and 4, the plurality of illumination fibres 28 and collection fibre 30 forms a single ring of fibres. Thereby, the illumination fibres 28 and the collection fibres 30 contact each other and are in contact with the instrument lumen 26 (hollow tube 34) and/or the elongate body 16, in particular the strain relief layer 24. However, the invention is not limited thereto. The illumination fibres 28 and/or the collection fibres 30 may not be in contact with each other or with the instrument lumen 26 and the elongate body 16, but are arranged spaced apart.

The space between the illumination fibres 28 and collection fibres 30 may be filled with a filler material and/or an adhesive such as epoxy. The adhesive may be used to fixate the plurality of illumination fibres 28 and collection fibres 30 with respect to each other.

In the embodiment of Fig. 3, the illumination fibres 28 and the collection fibre 30 are alternatingly arranged within a ring of single fibres. In contrast thereto, the embodiment depicted in Fig. 4 includes the same features and characteristics as the embodiment depicted in Fig. 3, however the collection fibres 30 form a first section in the single ring of fibres, wherein the illumination fibres 28 form a second section in the single ring of fibres. The number of collection fibre 30 may be larger than the number of illumination fibres 28. Thus, the first section of the single ring of fibres may be larger than the second section of the single ring of fibres. For example, the second section in the single ring of fibres may extend over a quarter or a third of the ring of fibres.

As visible in Fig. 2, the plurality of illumination fibres 28 and collection fibres 30 may extend beyond the proximal end 20 of the elongate body 16 and can be connected to the analysis device 12. A distal end of the collection fibres 28 and the collection fibres 30 may coincide with the distal end 18 of the elongate body 16. In order to seal the distal end 18, a cap device 36 may be provided at the distal end 18. The cap device 36 may include an aperture 38 which is aligned with the instrument lumen 26 such that the elongate instrument 32 can be pushed out of the instrument lumen 26 and through the aperture 38 of the cap device 36.

The cap device 36 may also include an optical structure which is coupled to the illumination fibres 28 and/or the collection fibres 30. The optical structure may be a window through which light from the illumination fibres 28 can pass and/or light coming from the target site can be coupled into the collection fibres 30. The illumination fibres 28 and/or the collection fibre 30 may contact the window and/or can be spaced apart to the window. The illumination fibres 28 and/or the collection fibres 30 may be fixedly positioned with respect to the optical structure.

The optical structure may include one or more lenses and/or one or more optical filters. The one or more lenses may be configured to focus the light coming from the illumination fibres 28 onto the target site. The one or more lenses may also be configured to focus the light coming from the target site onto a distal end face of the collection fibres 30 such that the light may be coupled into the collection fibres 30. The one or more optical filters may be positioned before the distal end face of the collection fibres 30 and may be configured to filter out light having a wavelength identical to the light emitted by the illumination fibres 28, i.e. Rayleigh or elastically scattered light.

The plurality of illumination fibres 28 and collection fibres 30 form a probe for a Raman spectroscopy measurements. As commonly known, Raman spectroscopy includes illuminating a sample with monochromatic light while the inelastically back scattered light (or Raman scattered light) is analysed, for example using a spectrometer. The Raman scattered light has a different wavelength compared to the monochromatic light with which the sample is illuminated.

In the present case, the analysis device 12 includes a Raman light source 40 (such as one or more lasers) to which the illumination fibres 28 are coupled. Thus, the monochromatic light is directed to the target site via the illumination fibres 28. The Raman scattered light (the light inelastically scattered back by the target site) is coupled into the collection fibres 32 and fed to a spectrometer 42 arranged in the analysis device 12. The Raman scattered light is analysed using the spectrometer 42. The intensity and/or wavelength of the Raman scattered light is indicative of the molecules present within the target site. The analysis of the molecules identified using the spectrometer 42 may indicate the presence of cancerous or other degenerated tissue at the target site. It is also possible to use the Raman spectroscopy measurements to identified different types of tissues present at the target site. This analysis step may be done manually or by a processor in combination with a respective software which may also be included in the analysis device 12.

The elongate instrument 32 of the embodiment depicted in Figs. 1 to 6 is an endoscopic camera 44. The endoscopic or elongate camera 44 may include an image capturing device 46. The image capturing device 46 is configured to create a series of the electrical signals based on optical image which is projected on an optical sensor arranged within the image capturing device 46. The optical sensor may be arranged at or close to a distal end of the elongate camera 44. A wire may extend within the elongate camera 44 from image capturing device 46 (in particular the optical sensor) to a proximal end of the elongate camera 44for transmitting the electrical signals. The proximal end of the elongate camera 44 is connected to the display device 14. The display device 14 may include a processor to process the series of electrical signals generated by the image capturing device 46. In particular, the processor may generate an optical image that can be displayed by a display 48 of the display device 14.

Thus, it is possible with the assembly shown in Figs. 1 to 6 to simultaneously image a target site while performing Raman spectroscopy measurements. This may help to visually identify areas of the target site which are cancerous, in particular in cases in which the cancerous tissue can only be detected using Raman spectroscopy measurements but not by using a camera.

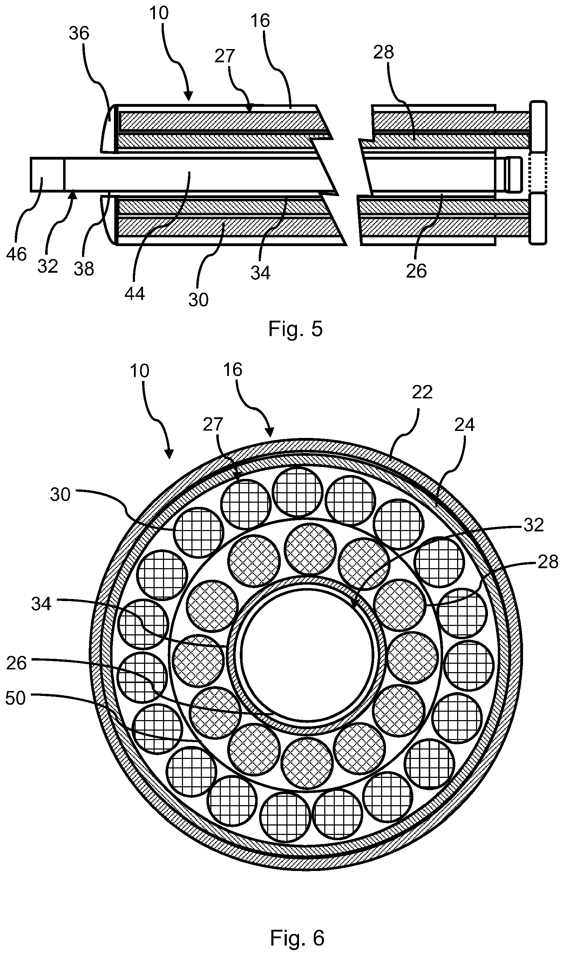

Figs. 5 and 6 refer to another embodiment of the Raman spectroscopy probe 10 having the same features and characteristics as the embodiment depicted in Figs. 1 to 4 except of the following differences.

The plurality of illumination fibres 28 is arranged in a first ring of fibres, while the plurality of collection fibres 30 is arranged in a second ring of fibres. In each ring, the plurality of collection fibres 30 and illumination fibres 28 are densely packed and may be contact each other. The first ring of collection fibres 30 is coaxially arranged with the second ring of illumination fibres 28 and, optionally, coaxially with the instrument lumen 26 and/or the elongate body 16. As depicted in Fig. 6, the second ring of collection fibres 30 is separated by the first ring of illumination fibres 28 by a middle wall 50. However, the middle wall 50 is not essential and can be omitted (see Fig. 5). The middle wall 50 may be constituted by a hollow tube and can be made from a plastic material. The middle wall 50 may be coaxially arranged with the instrument lumen 26, the hollow tube 34 and/or the elongate body 16.

The second ring of collection fibres 30 surrounds the first ring of illumination fibres 28. Thus, the cross-sectional area covered by the collection fibres 30 is greater than a cross-sectional area covered by the illumination fibres. This may contribute to a collection of as much as Rayleigh scattered light from the target site. For example, this arrangement results in that the number of collection fibres 30 is greater than the number of illumination fibres. Alternatively or additionally, the diameter of the collection fibres 30 may be greater than the diameter of the illumination fibres 28.

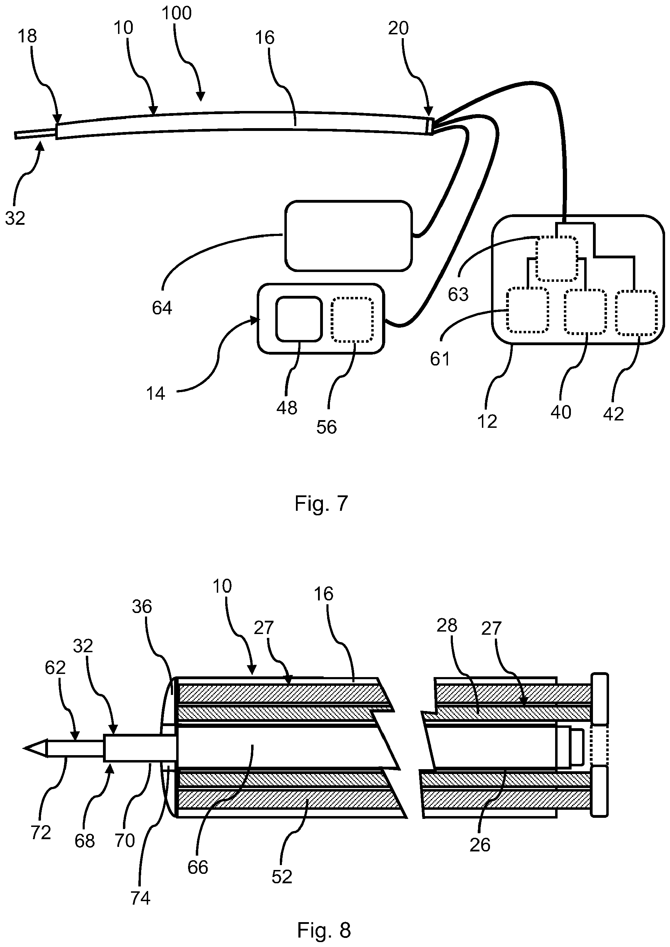

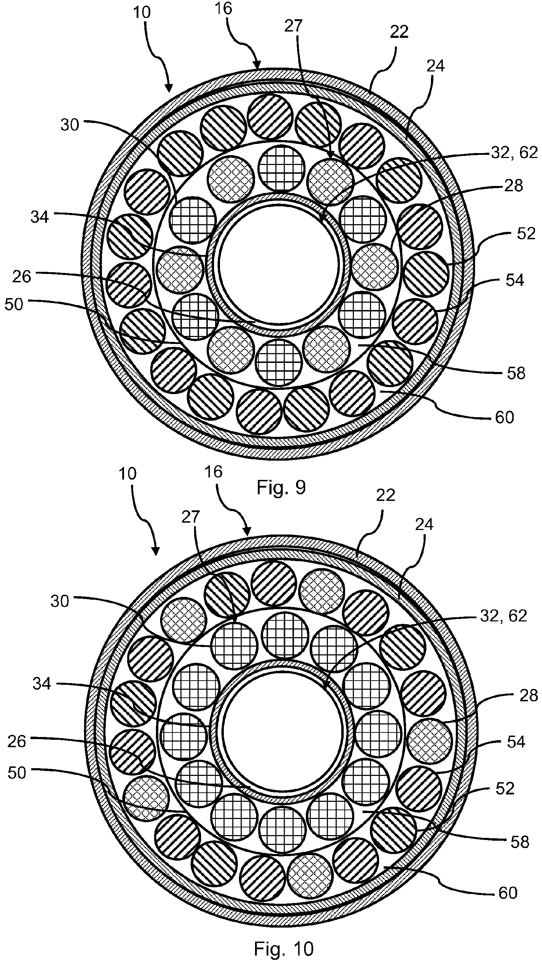

Figs. 7 to 9 depict another assembly including the Raman spectroscopy probe 10 according to another embodiment which is identical to the Raman spectroscopy probe of the embodiment depicted in figures 1 to 6 except for the following differences:
As visible in Figs. 8 and 9, the Raman spectroscopy probe 10 includes at least one imaging fibre 52 and/or at least one camera illumination fibre 54. The imaging fibres 52 and the camera illumination fibres 54 may be called camera fibres since they are part of another embodiment of the camera 44 to image to target site.

The imaging fibres 52 and the camera illumination fibres 54 extend beyond the proximal and 20 of the elongate body 16 and are connected to the display device 14.

The display device 14 may include a camera light source 56 which is configured to generate light such as white light and in particular non-monochromatic light. The camera illumination fibres 54 are connected to the camera light source 56. The camera illumination fibres 54 guide the light generated by the camera light source 56 to a distal end of the camera illumination fibres 54 which may coincide with the distal end 18 of the elongate body 16.

The camera illumination fibres 54 may be coupled to the optical structure of the cap device 36. For example, the cap device 36 may include one or more additional lenses for focusing (directing) the light emitted by the camera illumination fibres 54 onto the target site.

The optical structure of the cap device 56 may also include one or more lenses for coupling the light coming from the target site into the imaging fibres 52. For example, the light coupled into the imaging fibres may be that light which is shown onto the target site by the camera illumination fibres 54. The imaging fibres 52 guide the light from the distal end to the proximal and of the imaging fibres 52 which can be coupled to an optical sensor such as a CCD sensor. In particular, the light guided by the imaging fibres 52 is projected onto the optical sensor in such a way that an image of the target site is projected onto the optical sensor. The optical sensor may convert the impinging light beamed into a serious of electrical pulses which may be processed by a processor such that an image of the target site may be visible on the display 48.

In short, the imaging fibres 52 in conjunction with the optical sensor arranged in the display device 14 constitute a camera for imaging the target site. The camera illumination fibres 54 coupled to the camera light source 56 may be used to illuminate the target site. However, the camera illumination fibres 54 and the camera light source 56 are not mandatory as the target site may be illuminated by the light emitted by the illumination fibres 28. Even if monochromatic light is emitted by the illumination fibres 28, this might be sufficient for imaging the target site.

As better visible in Fig. 9, the illumination fibres 28 and the collection fibres 30 form a single ring of fibres while the imaging fibres 52 and the camera illumination fibres 54 form another single ring of fibres. The single ring of illumination fibres 28 and of collection fibres 30 may be coaxially arranged with the single ring of imaging fibres 52 and camera illumination fibres 54. In the embodiment depicted in Fig. 9, the single ring of imaging fibres 52 and camera illumination fibres 54 surrounds the single ring of illumination fibres 28 and collection fibres 30. However, the opposite arrangement is also possible which might has the advantage that the number of collection fibres 30 is increased compared to the embodiment depicted in Fig. 9. Thus, more light may be collected for the Raman spectroscopy measurements.

The imaging fibres 52 and the camera illumination fibres 54 are alternatingly arranged in the single ring of fibres. However, the imaging fibres 52 may form a section in the single ring of fibres while the camera illumination fibres 54 form another section in the single ring of fibres - similar to the sectional arrangement of the illumination fibres 28 and collection fibres 30 depicted in Fig. 4. It is also possible that the illumination fibres 28 and the collection fibres 30 are not alternatingly arranged step depicted in fig. 9 but in a sectoral arrangement as depicted in fig. 4.

The imaging fibres 52 and the camera illumination fibres 54 on the one hand are physically separated from the illumination fibres 28 and the collection fibres 30 of the other hand by middle wall 50. However, the middle wall 50 is not essential and can be omitted.

The middle wall 50 further divides the space within the elongate body 16 in a second lumen 58 and a third lumen 60. The plurality of illumination fibres 28 and collection fibres 30 are arranged in the second lumen while the plurality of imaging fibres 52 and camera illumination fibres 54 are arranged in the third lumen. In the embodiment depicted in fig. 9, the second lumen 58 and/or the third lumen 60 completely surround the instrument lumen 26. Optionally, the second lumen 58 and/or the third lumen 60 are coaxially arranged with the instrument lumen 26.

The display device 12 may additionally include an illumination source 61 and an optical illumination component 63. The illumination source 61 may be configured to generate non-monochromatic light, for example white light. The illumination source 61 may be have the same features and/or characteristics as the camera light source 56. The illumination source 61 may be coupled to the illumination fibres 28.

The illumination component 63 may be an optical switch in one embodiment, for example a single-pole-double-pole switch. The illumination component 63 may be configured to selectively switch the light source that is inputted into the illumination fibre 28. For example, when performing Raman spectroscopy measurements, the illumination component 63 couples the light of the Raman light source 40 into the illumination fibre 28. When imaging the target site using the elongate camera 44, the illumination component 63 couples the light from the illumination source 61 into the illumination fibre 28. It may also be possible that the illumination component 63 couples both the light from the Raman light source 40 and the light from the illumination source 61 into the illumination fibre 28 - for example when imaging and Raman spectroscopy measurements are simultaneously performed.

In another embodiment, the illumination component 63 may be an optical coupler which permanently couples the lights from both the Raman light source 40 and the illumination source 61 into the illumination fibre 28. The optical coupler may be a Y-branch coupler. Depending on the function to be performed, the Raman light source 40 and/or the illumination source 61 are selectively switched on or off.

The illumination component 63, the Raman light source 40 and the illumination source 61 may be connected to each other by waveguides and/or optical fibres.

The illumination source 61 and an optical illumination component 63 may be provided additionally or alternatively to the camera light source 56. In particular, the camera illumination fibres 54 may be omitted if the illumination source 61 and an optical illumination component 63 are provided. Thus, the illumination source 61 and an optical illumination component 63 provide an additional or alternative illumination of the target site for imaging by using the illumination fibres 28.

The elongate instrument 32 depicted in Figs. 7 and 8 is an electrosurgical treatment device 62 for emitting electromagnetic radiation such as a radio frequency radiation and/or microwave radiation which may be used for cutting, ablating, stimulating and/or coagulating tissue at the target site.

A proximal end of the treatment device 62 may be connected to a generator 64 which is capable of generating electromagnetic energy having frequencies in the radio frequency range and/or the microwave range.

The treatment device 62 may include a flexible transmission line 66 (such as a coaxial cable) and a radiating element 68 which is connected at a distal end of the transmission line 66. The transmission line 66 may be a conventional flexible 50 Ω coaxial cable suitable for conveying microwave energy. The transmission line 66 may include a centre conductor and an outer conductor that are separated by a dielectric material. The transmission line 66 is connectable at a proximal end to a generator, e.g. the generator 64, to receive the electromagnetic energy, in particular microwave energy.

The radiating element 68 includes a proximal coaxial transmission line 70 and a distal needle tip 72 formed at a distal end of the proximal coaxial transmission line 70. The proximal coaxial transmission line 70 is electrically connected to the distal end of the transmission line 66 to receive the electromagnetic energy from the transmission line 66 and convey it to the distal needle tip 72. The distal needle tip 72 is configured to deliver the received electromagnetic energy into biological tissue at the target site. In the present example, the distal needle tip 72 is configured as a half wavelength transformer to deliver microwave energy into target biological tissue, to ablate the target tissue. In other words, an electrical length of the distal needle tip 72 corresponds to a half wavelength of the microwave energy (e.g. at 5.8 GHz). When microwave energy is delivered to the distal needle tip 72, it may radiate the microwave energy along its length into surrounding biological tissue.

An inner conductor of the proximal coaxial transmission line 70 is electrically connected to the centre conductor of the transmission line 66. The radiating element 68 is secured to the transmission line 66 via a collar 74 mounted over a junction between the transmission line 66 and the radiating element 68. The collar 74 is made of a conductive material (e.g. brass), and electrically connects the outer conductor of the transmission line 66 to an outer conductor of the proximal coaxial transmission line 70. The outer conductor is formed of a tube of nitinol, which is flexible and provides a sufficient longitudinal rigidity to pierce tissue (e.g. the duodenum wall). For illustration purposes, the outer conductor is omitted from Fig. 8. Also for illustration purposes, a length of the proximal coaxial transmission line 70 has been omitted in Fig. 8. Exemplary details of the transmission line 66 and the radiating element 68 may be gathered from WO 2020/221749.

The transmission line 66 is fixedly arranged within the instrument lumen 26. For example, the transmission line 66 is adhered by means of an adhesive to the hollow tube 34. However, the hollow tube 34 may be omitted in the embodiment depicted in Figs. 7 to 9 since a sealing of the instrument lumen 26 is no longer necessary as the transmission line 66 is arranged within the elongate body 16. For example, an outer sheath of the transmission line 66 may replace the hollow tube 34. The plurality of illumination fibres 28 and collection fibres 30 may be fixed to the transmission line 66.

The collar 74 may form a part of the cap device 36. In such an embodiment, the cap device 36 combines the functionalities of the optical structure as described above with the electrical connection between the transmission line 66 and the radiating element 68. Additionally, the diameter of the aperture 38 is slightly larger than the outer diameter of the radiating element 68 but smaller than the outer diameter of the transmission line 66. Thus, the cap device 36 may cover parts of the transmission line 66 at the distal end 18. Optionally, a distal end of the transmission line 66 coincides with the distal end 18 of the elongate body 16. Thus, solely the radiating element 68 protrudes from the distal end 18 of the elongate body 16.

In an alternative embodiment not shown in the figures, the transmission line 66 includes a hollow inner conductor. The radiating element 68 may be configured to be inserted into the hollow inner conductor of the transmission line 66.

Alternatively or additionally, the treatment device 62 is not permanently fixed in the instrument lumen 26 and may be slidable within the instrument lumen 26. Thus, during navigation of the Raman spectroscopy probe 10 to the target site, the complete treatment device 62, in particular the radiating element 68, may not protrude from the distal end 18 of the elongate body 16 but is arranged within the instrument lumen 26. For emitting electromagnetic radiation, the radiating element 68 is pushed out of the distal end 18 of the elongate body 16.

The Raman spectroscopy probe 10 of Figs. 7 to 9 may combine the functionalities of a camera, of a Raman spectroscopy device and of a surgical treatment device. In particular, it is possible to identify tissue to be treated at the target site using the camera 44 (including the imaging fibres 52 and the camera illumination fibre 54) and analyse the tissue using the Raman spectroscopy capabilities provided by the illumination fibre 28 and the collection fibre 30. Monitoring of the target site and the analysis of the tissue at the target site may be continued during the application of electromagnetic energy by the treatment device 62. Thus, it is possible to image the target site and to identify cancerous tissue while ablating tissue at the target site.

The embodiment of the Raman spectroscopy probe 10 depicted Fig. 10 has the same features and functionalities as the embodiment of the Raman spectroscopy probe 10 depicted in Figs. 7 to 9 except for the following differences:
The collection fibres 30 may form a complete ring of fibres distributed around the instrument lumen 26. In particular, only collection fibres 30 are arranged in the second lumen 58. No other types of fibres are arranged in the second lumen 58. The at least one illumination fibre 28 can be arranged in the third lumen 60. Thus, the at least one illumination fibre 28 is arranged radially outside of the ring of collection fibres 30. The imaging fibres 52 and/or the camera illumination fibres 54 may be arranged in the third lumen 60. However, the middle wall 50 separating the third lumen 60 from the second lumen 58 may be omitted while maintaining the arrangement of the different types of fibres. The illumination fibres 28, the imaging fibres 52 and/or the camera illumination fibres 54 may form a single ring of fibres which surrounds the ring of fibres constituted by the plurality of collection fibres 30.

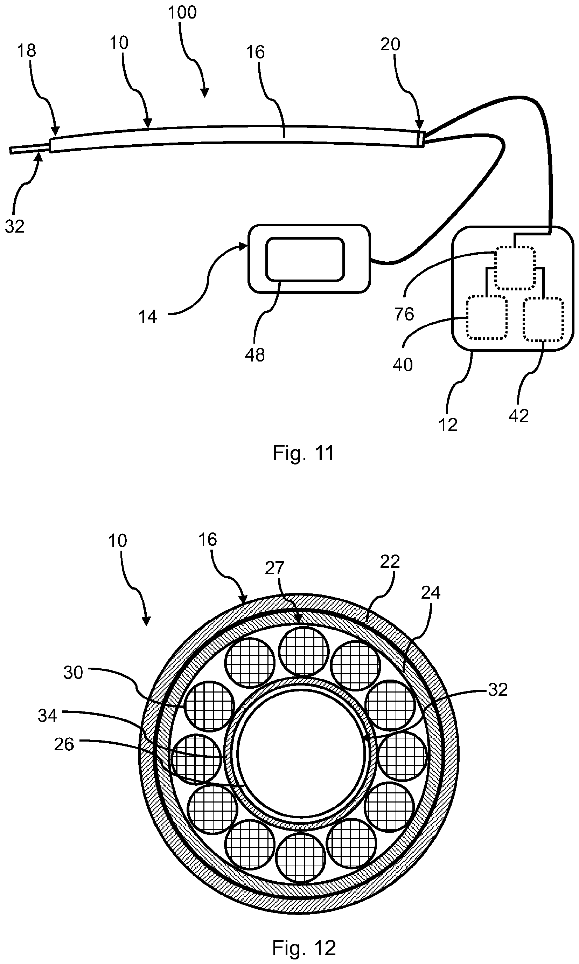

The embodiment of the Raman spectroscopy apparatus 100 including the Raman spectroscopy probe 10 according to Figs. 11 and 12 has the same features and functionalities as the embodiment of the Raman spectroscopy apparatus 100 including the Raman spectroscopy probe 10 depicted in Figs. 1 to 3 except for the following differences:
The Raman spectroscopy probe 10 includes only one type of Raman fibres 27, for example only collection fibres 30. The collection fibres 30 may be additionally configured to direct light from the proximal end 20 to the distal end 18. The Raman fibres 27 are arranged in a single ring of fibres around the instrument lumen 26. Thus, the same Raman fibres 27 are used for guiding light from the proximal end 20 to the distal end 18 as well as from the distal end 18 to the proximal end 20.

The analysis device 12 further includes an optical detection component 76 to which the Raman fibres 27 are coupled to. The optical detection component 76 is further connected to both the Raman light source 40 and the spectrometer, for example by waveguides and/or optical fibres.

The optical detection component 76 may include a time division switch and/or a multiplexer. The optical detection component 76 is configured to route light depending on the wavelength, polarization, and/or phase from the Raman light source 40 into the Raman fibres 27 and/or from the Raman fibres 27 to the spectrometer 42. This selective routing helps to reduce/filter out Rayleigh scattered light from the Raman scattered light. However, the optical detection component 76 may include a coupler and, optionally, some optical filters.

The optical detection component 76 couples light from the Raman light source 40 into the Raman fibre 27. In addition, the optical detection component 76 directs Raman scattered light to the spectrometer 72. This functionality may be selectively performed, for example temporally selective or depending on the respective wavelength. The optical detection component 76 allows to reduce the number of Raman fibres 27 or increase the number of fibres which act as collection fibres 30, since each fibre acts as a collection fibres 30.

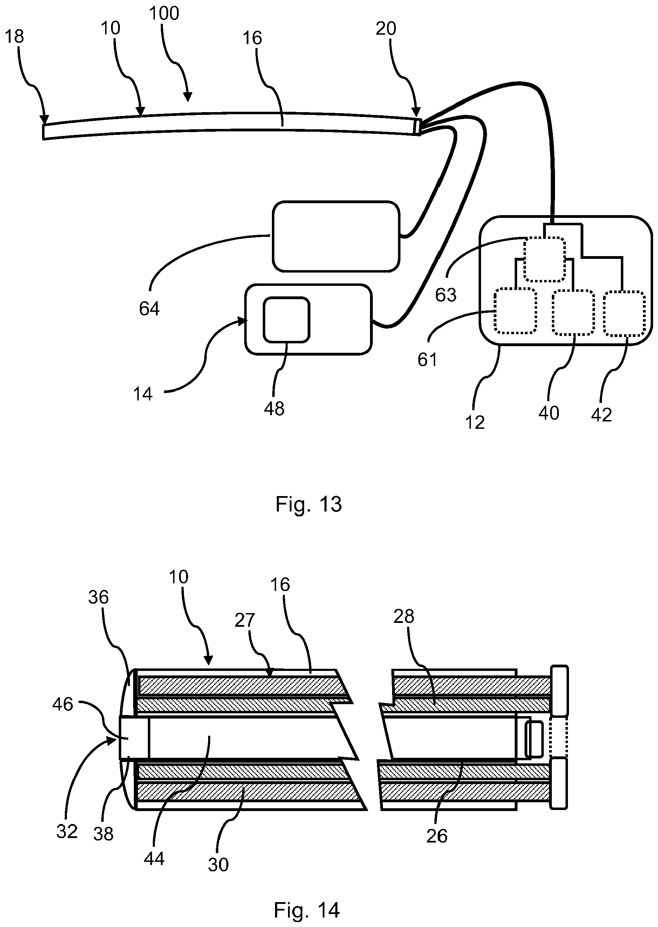

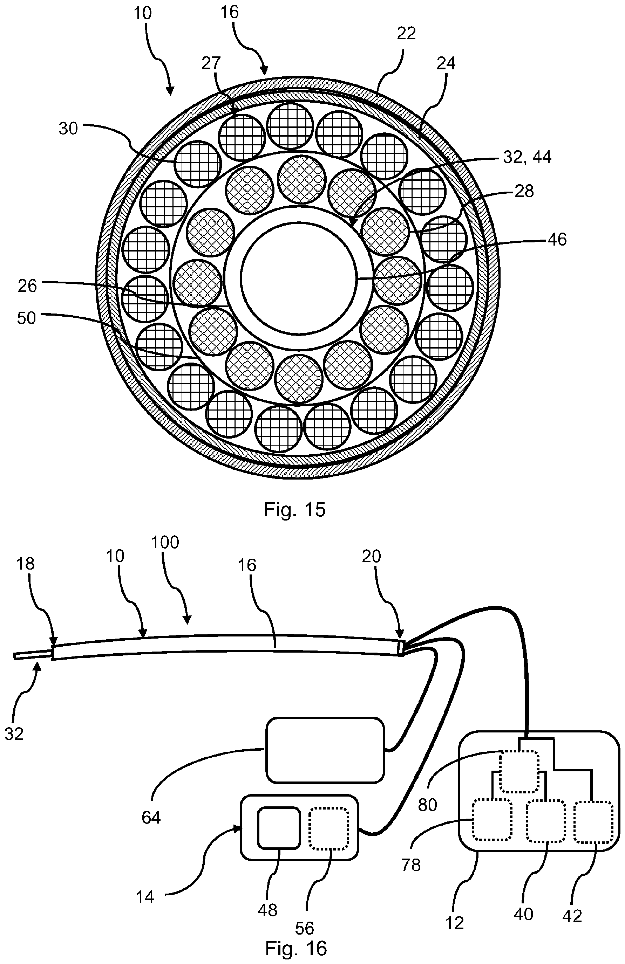

The embodiment of the Raman spectroscopy apparatus 100 including the Raman spectroscopy probe 10 according to Figs. 13 and 15 has the same features and functionalities as the embodiment of the Raman spectroscopy apparatus 100 including the Raman spectroscopy probe 10 depicted in Figs. 7 to 9 except for the following differences:
The elongate instrument 32 is a camera 44 which is permanently fixed in the instrument lumen 26. A distal end of the camera 44 coincides with the distal end 18 of the elongate body 16. The hollow tube 34 may be omitted. An outer surface of the camera 44 may be in direct contact with the Raman fibres 27.

The camera 44 includes the image capturing device 46. The camera 44 does not include means for illuminating the target site. The illumination of the target site is provided by means of the illumination source 61. The light generated by the illumination source 61 is coupled in the ring of illumination fibres 28 by the illumination component 63 which may be an optical switch or an optical coupler. The illumination fibres 28 are also used to guide the light generated by the Raman light source 40 to the target site.

The ring of illumination fibres 28 may surround the camera 44. The ring of illumination fibres 28 is surrounded by a ring of collection fibres 30. In an alternative embodiment not depicted in the figures, the camera 44 is surrounded by a ring of camera illumination fibres 54 which in turn is surrounded by a ring of illumination fibres 28 and a ring of collection fibres 30. In this embodiment, the illumination source 61 and the illumination component 63 may be omitted. Instead, the camera light source 56 is provided to which the camera illumination fibres 54 are coupled.

The embodiment of the Raman spectroscopy apparatus 100 including the Raman spectroscopy probe 10 according to Fig. 16 has the same features and functionalities as the embodiment of the Raman spectroscopy apparatus 100 including the Raman spectroscopy probe 10 depicted in Figs. 13 to 15 except for the following differences:
The Raman spectroscopy apparatus 100 further includes a treatment light source 78 and an optical coupler component 80. The illumination source 61 and the illumination component are not present with the Raman spectroscopy apparatus 100 of Fig. 16. The treatment light source 78 and the optical coupler component 80 may be arranged within the analysis device 12 (as depicted) or external to the analysis device 12.

The treatment light source 78 is configured to generate light having an intensity sufficiently high to modify, in particular cut, ablate and/or coagulate, tissue, in particular soft tissue. The intensity of the light generated by the treatment light source 78 may be solely sufficiently high to modify tissue if the light is focused on a small volume, for example using a convex lens or focusing lens system. The treatment light source 78 may include one or more lasers, in particular semiconductor lasers.

The light generated by the treatment light source 78 is coupled into an input of the optical coupler component 80. Another input of the optical coupler component 80 is coupled to the Raman light source 40. The optical coupler component 80 may have the same characteristics and/or features as the illumination component 63 described above. In particular, the optical coupler component 80 is configured to couple the light received at the inputs into a single output which may be coupled to the Raman fibre(s) 27, in particular to the illumination fibre(s) 28. The optical coupler component 80 allows that light generated by the Raman light source 40 and/or the treatment light source 78 is coupled into the Raman fibre 27 and can propagate to the distal end 18, in other words to the target site.

The light generated by the Raman light source 40 may be used to detect cancerous tissue as described above. When analysing the cancerous tissue using Raman spectroscopy, the optical coupler component 80 is configured to couple the light from the Raman light source 40 into the Raman fibre 27. Once the Raman spectroscopy measurement is finished, the optical coupler component 80 is switched to couple the light from the treatment light source 78 into the Raman fibre 27 in order to modify the cancerous tissue, for example to cut the cancerous tissue from healthy tissue. Alternatively, the optical coupler component 80 may be configured to simultaneously couple the light from the Raman source 40 and the treatment light source 78 into the Raman fibre 27. In this case, the Raman spectroscopy measurements may be performed while cutting tissue. This embodiment may be applied if the wavelength of the light generated by the treatment light source 78 does not affect the Raman spectroscopy measurement.

Optical filters (e.g. band pass filters or wavelength dependent filters) may be provided which are configured to suppress backscattered light which otherwise would be impinge into the Raman light source 40, the treatment light source 78 and/or illumination source 61. Such backscattered light may affect the functioning of the light source or lasers.

In another embodiment (in view of the embodiment of Figs. 7 and 13), one input of the optical coupler component 80 may by coupled to the illumination source 61 and the other input of optical coupler component 80 may by coupled to the treatment light source 78. In this embodiment which is not depicted in the figures, the output of the optical coupler component 80 is coupled to the at least one camera fibre, in particular to the at least one camera illumination fibre 54.

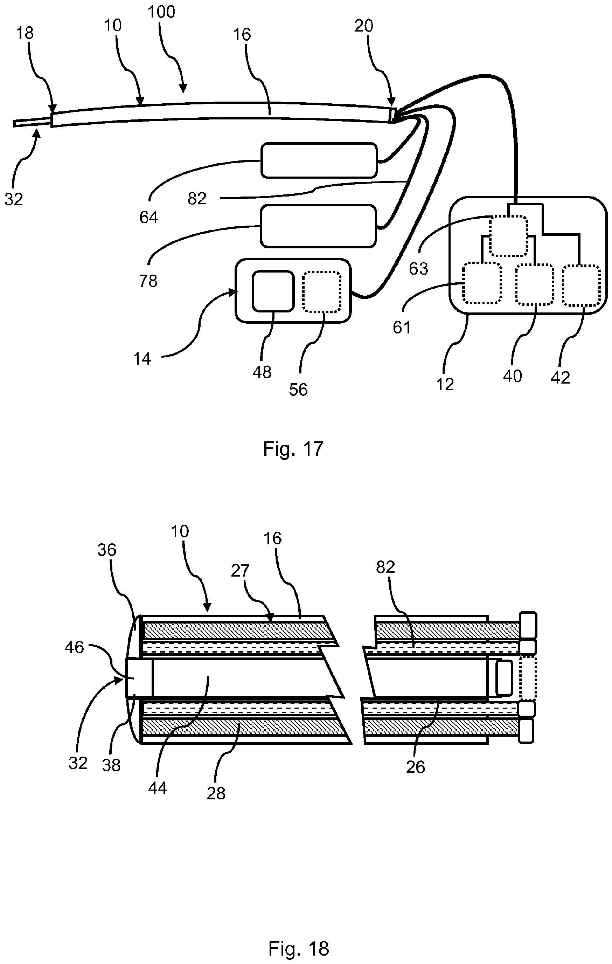

The embodiment of the Raman spectroscopy apparatus 100 including the Raman spectroscopy probe 10 according to Figs. 17 to 19 has the same features and functionalities as the embodiment of the Raman spectroscopy apparatus 100 including the Raman spectroscopy probe 10 depicted in Figs. 13 to 15 except for the following differences:
The Raman spectroscopy apparatus 100 additionally includes the treatment light source 78 (optionally having the characteristics and/or features as described in conjunction with the embodiment of Fig. 16) and at least one treatment fibre 82 which is coupled to the treatment light source 78.

The at least one treatment fibre 82 extends from the treatment light source 78 through the elongate body 16 to the distal end 18 of the elongate body 16. The at least one treatment fibre 82 is arranged within the elongate body 16, but outside of the instrument lumen 26 (see Fig. 18). The at least one treatment fibre 82 may be arranged between the plurality of Raman fibres 27 and camera fibres. In case of a plurality of treatment fibres 82, they can be arranged in configurations similar to the ones of the Raman fibres 27 and/or camera fibres.

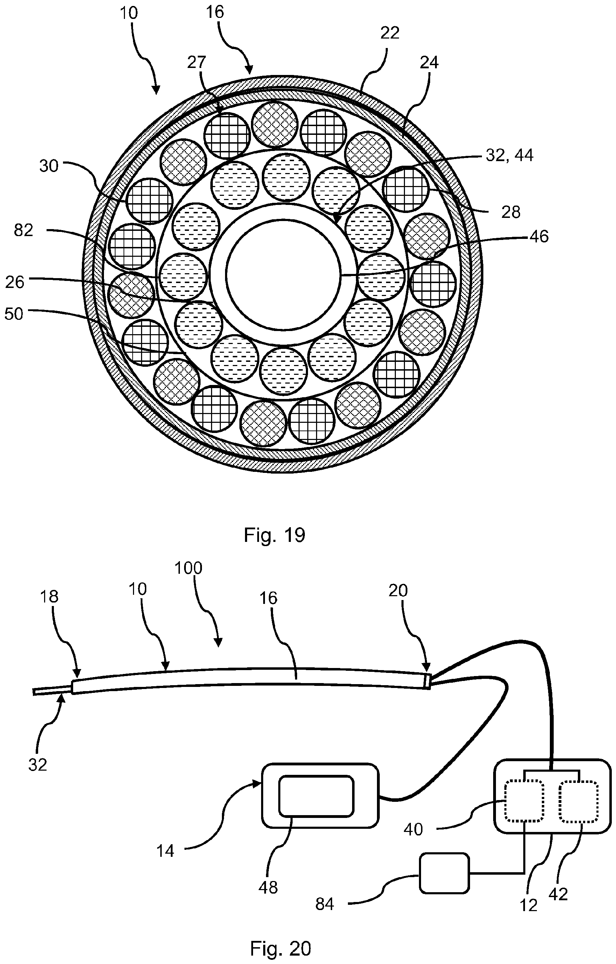

As visible in Figs. 18 and 19, the treatment fibres 82 form a ring of fibres around the instrument lumen 26. The Raman fibres 27 form another ring of fibres which surround the ring of treatment fibres 82. The treatment fibres 82 may include a lens or lens system at the distal end 18 for focussing the light emitted at the distal end 18 onto the target site. Alternatively, as depicted in Fig. 18, the treatment fibres 82 are coupled to the cap device 36 which includes optics for focussing the light emitted at the distal end 18 of the treatment fibres 82 onto the target site.

The embodiment of the Raman spectroscopy apparatus 100 including the Raman spectroscopy probe 10 according to Fig. 20 has the same features and functionalities as the embodiment of the Raman spectroscopy apparatus 100 including the Raman spectroscopy probe 10 depicted in Figs. 1 to 3 except for the following differences:
The Raman spectroscopy apparatus 100 further includes a controller 84 which is connected to the Raman light source 40 which, at least in this embodiment, is configured to emit light of different intensities. In particular, the Raman light source 40 is configured generate light having a maximum intensity which corresponds to the intensity of the light generated by the treatment light source 78 discussed with the embodiments of Figs. 16 to 19.

In the embodiment of Fig. 20, the Raman light source 40 additionally provides the capabilities of the treatment light source 78. When performing a Raman spectroscopy measurement, the controller 84 controls the intensity of the light generated by the Raman light source 40 to such a level which is suitable for Raman measurements. When modifying (cutting) tissue at the target site, the controller 84 increases the intensity of the light generated by the Raman light source 40 to such a level which facilitates the modification of tissue. In particular, the controller 84 increases the intensity of the light generated by the Raman light source 40 to a maximum level. For example, the power of the light for Raman spectroscopy could be 25mW which may be increased to 25W for modifying the tissue.

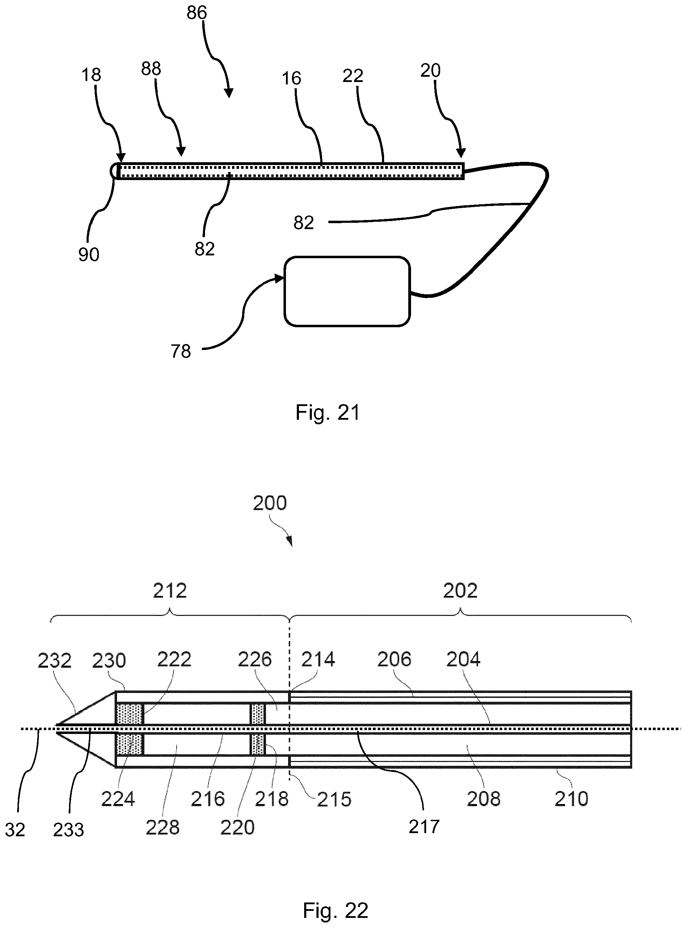

Fig. 21 depicts an embodiment of a treatment apparatus 86 which includes the treatment light source 78 and a treatment probe 88. The treatment light source 78 may have the same characteristics as described above. The treatment probe 88 includes an elongate body 16 and at least one treatment fibre 82 which may be similar to the one described above.

The treatment fibre 82 extends from the distal end 18 of the elongate body 16 to the proximal end 20 of the elongate body 16. The treatment fibre 82 protrudes from the proximal end 20 of the elongate body 16 and is coupled to the treatment light source 78. The elongate body 16 may include a sheath 22 within which the treatment fibre 82 extends. Further components except of the at least one treatment fibre 82 may not be arranged within the sheath 22 which may have the same characteristics as described above. For example, the treatment probe 88 is free of an instrument lumen or optical fibres not coupled to the treatment light source 78. A treatment lens 90 may be provided at the distal end 18. The treatment lens 90 may be configures to focus the light emitted by the treatment fibre 82 at the distal end 18 onto the target site. The treatment lens 90 may include one or more lenses, in particular convex lenses.

The treatment probe 88 of the treatment apparatus 86 may be inserted into the instrument lumen 26 of the Raman spectroscopy probe 10. Alternatively, the treatment apparatus 86 may be fixed to the instrument lumen 26 of the Raman spectroscopy probe 10.

Fig. 22 shows a cross-sectional side view of an elongate assembly 200. The elongate assembly 200 includes a coaxial feed cable 202 that is connectable at its proximal end to a generator (such as generator 64) in order to convey microwave energy. The coaxial feed cable 202 comprises an inner conductor 204 and an outer conductor 206 which are separated by a dielectric material 208. The coaxial feed cable 202 is preferably low loss for microwave energy. A choke (not shown) may be provided on the coaxial feed cable 202 to inhibit back propagation of microwave energy reflected from the distal end and therefore limit backward heating along the device. The coaxial feed cable 202 further includes a flexible outer sheath 210 disposed around the outer conductor 206 to protect the coaxial feed cable 204. The outer sheath 210 may be made of an insulating material to electrically isolate the outer conductor 206 from its surroundings. The outer sheath 210 as well as the outer sheath 22 may be made of, or coated with, a non-stick material such as PTFE to reduce friction.

A radiating tip 212 is formed at the distal end 214 of the coaxial feed cable 202. The dashed line 215 in Fig. 22 illustrates an interface between the coaxial feed cable 202 and the radiating tip 212. The radiating tip 212 is arranged to receive microwave energy conveyed by the coaxial feed cable 202, and deliver the energy into biological tissue, for example at the target site. The outer conductor 206 of the coaxial feed cable 202 terminates at the distal end 214 of the coaxial feed cable 202, i.e. the outer conductor 206 does not extend into the radiating tip 212. The radiating tip 212 includes a distal portion 216 of the inner conductor 204 which extends beyond the distal end of the coaxial feed cable 202. In particular, the distal portion 216 of the inner conductor 204 extends beyond a distal end of the outer conductor 206.

The inner conductor 204 of the coaxial feed cable 202 and the inner conductor 216 of the radiating tip 212 are hollow to form a passageway 217 into which the elongate instrument 32 may be inserted. The inner diameter of the passageway 217 may be 1.8 mm to 2.0 mm, while the outer diameter of the elongate instrument 32 is slightly smaller to allow for a clearance gap.

A proximal tuning element 218 (or first tuning element) made of a conductive material (e.g. metal) is electrically connected to the distal portion 216 of the inner conductor 204 near a proximal end of the radiating tip 212. The proximal tuning element 218 has a cylindrical shape, and includes a channel 220 through which the distal portion 216 of the inner conductor 204 passes. A diameter of the channel 220 is substantially the same as an outer diameter of the inner conductor 204, such that the inner conductor 204 is in contact with the proximal tuning element 218 inside the channel 220. The proximal tuning element 218 may be further secured to the inner conductor 204, e.g. using a conductive adhesive (e.g. conductive epoxy) or by soldering or welding. The proximal tuning element 218 is centred on the inner conductor 204. In other words, a central axis of the cylindrical proximal tuning element 218 is collinear with the longitudinal axis of the inner conductor 204. In this manner, the proximal tuning element 218 is disposed around the distal portion 216 of the inner conductor 204 in a manner that is symmetrical about the longitudinal axis of the inner conductor 204.

An optional distal tuning element 222 (or second tuning element) made of a conductive material (e.g. metal) is electrically connected to the distal portion 216 of the inner conductor 204 near a distal end of the radiating tip 212. Thus, the distal tuning element 222 is located further along the inner conductor 204 than the proximal tuning element 218. The distal tuning element 222 is spaced apart from the proximal tuning element by a length of the distal portion 216 of the inner conductor 204. Like the proximal tuning element 218, the distal tuning element has a cylindrical shape and includes a channel 224. As can be seen in Fig. 22, the distal portion 216 of the inner conductor 204 extends into the channel 224. The distal portion 216 of the inner conductor 204 terminates at a distal end of the channel 224, i.e. it does not protrude beyond the distal tuning element 222. In this manner, a distal end of the inner conductor 204 lies flush with a distal face of the distal tuning element 222. A diameter of the channel 224 is substantially the same as the outer diameter of the inner conductor 204, such that the inner conductor 204 is in contact with the distal tuning element 222 inside the channel 224. The distal tuning element 222 may be further secured to the inner conductor 204, e.g. using a conductive adhesive (e.g. conductive epoxy) or by soldering or welding. Like the proximal tuning element 218, the distal tuning element 222 is mounted so that it is centred on the inner conductor 204. Both the proximal tuning element 218 and the distal tuning element 222 have the same outer diameter. The outer diameter of the proximal tuning element 218 and the distal tuning element 222 may be slightly less than the outer diameter of the electrosurgical instrument 200. In the example shown, the distal tuning element 222 is longer than the proximal tuning element 218 in the longitudinal direction of the instrument. In other words, the length of inner conductor 204 in channel 224 in the distal tuning element 222 is greater than the length of inner conductor 204 in channel 220 in the proximal tuning element 218. For example, the distal tuning element 222 may be approximately twice as long as the proximal tuning element 218. By making the distal tuning element 222 longer than the proximal tuning element 218, it is possible to concentrate microwave emission around the distal end of the radiating tip 212.

A distal portion 226 of the dielectric material 208 extends beyond the distal end 214 of the coaxial feed cable 202 into the radiating tip 212. The distal portion 226 of the dielectric material 208 acts as a spacer between the proximal tuning element 218 and the distal end 214 of the coaxial feed cable 202. In some embodiments (not shown), the dielectric material 208 may terminate at the distal end 214 of the coaxial feed cable 202, and a separate spacer may be provided between the distal end 214 of the coaxial feed cable 202 and the proximal tuning element 218. A dielectric spacer 228 is provided in the radiating tip 212 between the proximal tuning element 218 and the distal tuning element 222. The dielectric spacer 228 is a cylindrical piece of dielectric material, having a central channel extending therethrough. Thus, the dielectric spacer 228 may be a tube of dielectric material. The distal portion 214 of the inner conductor 204 extends through the channel in the dielectric spacer 228. A proximal face of the dielectric spacer 228 is in contact with the proximal tuning element 218, and a distal face of the dielectric spacer 228 is in contact with the distal tuning element 222. The dielectric spacer 228 has approximately the same outer diameter as the proximal and distal tuning elements 218, 222.

A protective sheath 230 is provided on the outside of the radiating tip 212. The protective sheath 230 covers the dielectric spacer 228 and the proximal and distal tuning elements 218, 222 to form an outer surface of the radiating tip 212. The protective sheath 230 may be a tube made of an insulating material. The protective sheath 230 may serve to insulate the radiating tip 212 and protect it from the environment. The protective sheath 230 may be made of or coated with a non-stick material (e.g. PTFE) to prevent tissue from sticking to it. An outer diameter of the protective sheath 230 is substantially the same as the outer diameter of the coaxial feed cable 202, so that the instrument has a smooth outer surface, i.e. the radiating tip 212 has an outer surface that is flush with an outer surface of the coaxial feed cable 202 at the interface 215. In some embodiments (not shown) the protective sheath 230 may be a continuation of the outer sheath 210 of the coaxial feed cable 202. Together, the distal portion 226 of the dielectric material 208, the dielectric spacer 228 and the protective sheath 230 form a dielectric body of the radiating tip 212.

The radiating tip 212 may further include a distal tip 232 located at its distal end. The distal tip 232 may be pointed in order to facilitate insertion of the radiating tip 212 into target tissue. However, in other embodiments (not shown), the distal tip may be rounded or flat. The distal tip 232 may be made of a dielectric material, e.g. the same as dielectric material 208. In some embodiments, the material of the distal tip 232 may be selected to improve impedance matching with target tissue, in order to improve the efficiency with which the EM energy is delivered to the target tissue. The distal tip 232 may be made of, or covered with a non-stick material (e.g. PTFE) to prevent tissue from sticking to it.

The distal tip 232 may include a through-hole 233 extending in the direction of the passageway 217 which is aligned with the passageway 217. The through-hole 233 has the same inner diameter as the passageway 217. The elongate instrument 32 may be pushed through passageway 217 and the through-hole 233 out of the elongate instrument 200.

The following are example dimensions of electrosurgical instrument 200:
- distance from the interface 215 to the distal end of the distal portion 216 of the inner conductor 204: 9.1mm;
- outer diameter of proximal tuning element 218 and distal tuning element 222: 2.4mm;
- length of proximal tuning element 218: 0.8 mm;
- length of distal tuning element 222: 1.6 mm;
- spacing between proximal tuning element 218 and distal tuning element 222: 5.9 mm;
- spacing between the proximal tuning element 218 and the interface 215: 0.8 mm; and
- outer diameter of electrosurgical instrument 200: 3.0 mm.

The radiating tip 212 may act as a microwave monopole antenna when microwave energy is conveyed to the radiating tip 212. In particular, microwave energy may be radiated from the distal portion 216 of the inner conductor 202, so that microwave energy can be delivered into surrounding biological tissue. The proximal and distal tuning elements 218, 222 act to shape the radiation profile of the radiating tip 212, and improve impedance matching between the instrument and surrounding target tissue, as discussed below.

The elongate instrument 32 that can be inserted into the passageway 217 may be a (endoscopic) surgical instrument, an endoscopic camera 44 or an elongate Raman spectroscopy instrument. The endoscopic camera 44 may be configured as described above. The elongate Raman spectroscopy instrument may include at least one illumination fibre 28, at least one collection fibre 30, the elongate body 16 and/or the cap device 36 which may have the configurations and figures as described above. In an example outside the scope of the current invention, the elongate Raman spectroscopy instrument may be configured as the Raman spectroscopy probe 10 without the instrument lumen 26.

## Claims

1. A Raman spectroscopy apparatus, comprising
the Raman spectroscopy probe (10),
an analysis device (12) including a spectrometer (72) and a Raman light source (40) for generating monochromatic light,
a treatment light source (78) configured to generate light having an intensity configured to modify tissue, and
an optical coupler component (80),
wherein the Raman spectroscopy probe (10) comprises
an elongate body (16) having a distal end (18) and a proximal end (20),
at least one Raman fibre (27) within the elongate body (16) for guiding light between the proximal end (20) and the distal end (18), and
an instrument lumen (26) within the elongate body (16) and extending between the distal end (18) and the proximal end (20),
wherein the instrument lumen (26) is configured to receive an elongate instrument (32),
wherein the at least one Raman fibre (27) is arranged outside of the instrument lumen (26),
wherein the at least one Raman fibre (27) is coupled to the analysis device (12), in particular to the Raman light source and/or the spectrometer and
wherein the optical coupler component (80) is configured to selectively or permanently couple the light from the Raman light source (40) and the light from the treatment light source (78) into the at least one Raman fibre (27), wherein inputs of the optical coupler component (80) are coupled to the Raman light source (40) and the treatment light source (78) respectively and the output of the optical coupler component (80) is coupled to the Raman fibre (27).

2. A Raman spectroscopy apparatus, comprising
the Raman spectroscopy probe (10),
an analysis device (12) including a spectrometer (72), a Raman light source (40) for generating monochromatic light, and an illumination source (61) for generating non-monochromatic light,
a treatment light source (78) configured to generate light having an intensity configured to modify tissue, and
an optical coupler component (80),
wherein the Raman spectroscopy probe (10) comprises
an elongate body (16) having a distal end (18) and a proximal end (20),
at least one Raman fibre (27) within the elongate body (16) for guiding light between the proximal end (20) and the distal end (18),
at least one camera illumination fibre (54) which is configured to guide light from the proximal end (18) to the distal end (20) and
an instrument lumen (26) within the elongate body (16) and extending between the distal end (18) and the proximal end (20),
wherein the instrument lumen (26) is configured to receive an elongate instrument (32),
wherein the at least one Raman fibre (27) is arranged outside of the instrument lumen (26),
wherein the at least one Raman fibre (27) is coupled to the analysis device (12), in particular to the Raman light source and/or the spectrometer and
wherein the optical coupler component (80) is configured to selectively or permanently couple the light from the illumination source (61) and the light from the treatment light source (78) into the at least one camera illumination fibre (54), wherein inputs of the optical coupler component (80) are coupled to the illumination source (61) and treatment light source (78) respectively and the output of the optical coupler component (80) is coupled to the camera illumination fibre (54).

3. The Raman spectroscopy apparatus according to any one of the claims 1 to 2, wherein the Raman light source (40) and/or the illumination source (61) are configured to vary an intensity of the generated light.

4. The Raman spectroscopy apparatus according to claim 3, wherein the Raman spectroscopy apparatus (100) further comprises a controller (84) for controlling the intensity of the light generated by the Raman light source (40) and/or the illumination source (61).

5. The Raman spectroscopy apparatus according to any one of the claims 1 to 4, wherein the analysis device (12) further includes an optical illumination component (63) and an illumination source (61) for generating non-monochromatic light, the optical illumination component (63) coupled to the at least one Raman fibre (27), the Raman light source (40) and the illumination source (61), wherein the optical illumination component (63) is configured to selectively or permanently couple the monochromatic light from the Raman light source (40) and/or the non-monochromatic light from the illumination source (61) into the at least one Raman fibre (27).

6. The Raman spectroscopy apparatus according to any one of the claims 1 to 5, wherein the analysis device (12) further includes an optical detection component (76), the optical detection component (76) coupled to the at least one Raman fibre (27), the Raman light source (40) and the spectrometer (72), wherein the optical detection component (76) is configured to route the monochromatic light of the Raman light source (40) into the at least one Raman fibre (27) and the light from the at least one Raman fibre (27) to the spectrometer (72).

## Patentansprüche

1. Raman-Spektroskopievorrichtung, umfassend:
die Raman-Spektroskopiesonde (10),
eine Analysevorrichtung (12), einschließlich eines Spektrometers (72) und einer Raman-Lichtquelle (40) zum Erzeugen von monochromatischem Licht,
eine Behandlungslichtquelle (78), die ausgelegt ist, um Licht mit einer Intensität zu erzeugen, die ausgelegt ist, um Gewebe zu modifizieren, und
eine Optische-Kopplerkomponente (80),
wobei die Raman-Spektroskopiesonde (10) Folgendes umfasst:
einen länglichen Körper (16), der ein distales Ende (18) und ein proximales Ende (20) aufweist,
zumindest eine Raman-Faser (27) innerhalb des länglichen Körpers (16) zum Führen von Licht zwischen dem proximalen Ende (20) und dem distalen Ende (18) und
ein Instrumentenlumen (26) innerhalb des länglichen Körpers (16), das sich zwischen dem distalen Ende (18) und dem proximalen Ende (20) erstreckt,
wobei das Instrumentenlumen (26) ausgelegt ist, um ein längliches Instrument (32) aufzunehmen,
wobei die zumindest eine Raman-Faser (27) außerhalb des Instrumentenlumens (26) angeordnet ist,
wobei die zumindest eine Raman-Faser (27) mit der Analysevorrichtung (12) gekoppelt ist, insbesondere mit der Raman-Lichtquelle und/oder dem Spektrometer und
wobei die Optische-Kopplerkomponente (80) ausgelegt ist, um das Licht aus der Raman-Lichtquelle (40) und das Licht aus der Behandlungslichtquelle (78) selektiv oder permanent in die zumindest eine Raman-Faser (27) zu koppeln, wobei Eingänge der Optische-Kopplerkomponente (80) mit der Raman-Lichtquelle (40) bzw. der Behandlungslichtquelle (78) gekoppelt sind, und der Ausgang der Optische-Kopplerkomponente (80) mit der Raman-Faser (27) gekoppelt ist.

2. Raman-Spektroskopievorrichtung, umfassend:
die Raman-Spektroskopiesonde (10),
eine Analysevorrichtung (12), einschließlich eines Spektrometers (72), einer Raman-Lichtquelle (40) zum Erzeugen von monochromatischem Licht, und einer Beleuchtungsquelle (61) zum Erzeugen von nichtmonochromatischem Licht,
eine Behandlungslichtquelle (78), die ausgelegt ist, um Licht mit einer Intensität zu erzeugen, die ausgelegt ist, um Gewebe zu modifizieren und
eine Optische-Kopplerkomponente (80),
wobei die Raman-Spektroskopiesonde (10) Folgendes umfasst:
einen länglichen Körper (16), der ein distales Ende (18) und ein proximales Ende (20) aufweist,
zumindest eine Raman-Faser (27) innerhalb des länglichen Körpers (16) zum Führen von Licht zwischen dem proximalen Ende (20) und dem distalen Ende (18),
zumindest eine Kamerabeleuchtungsfaser (54), die ausgelegt ist, um Licht von dem proximalen Ende (18) an das distale Ende (20) zu führen und
ein Instrumentenlumen (26) innerhalb des länglichen Körpers (16), das sich zwischen dem distalen Ende (18) und dem proximalen Ende (20) erstreckt,
wobei das Instrumentenlumen (26) ausgelegt ist, um ein längliches Instrument (32) aufzunehmen,
wobei die zumindest eine Raman-Faser (27) außerhalb des Instrumentenlumens (26) angeordnet ist,
wobei die zumindest eine Raman-Faser (27) mit der Analysevorrichtung (12) gekoppelt ist, insbesondere mit der Raman-Lichtquelle und/oder dem Spektrometer und
wobei die Optische-Kopplerkomponente (80) ausgelegt ist, um das Licht aus der Beleuchtungsquelle (61) und das Licht aus der Behandlungslichtquelle (78) selektiv oder permanent in die zumindest eine Kamerabeleuchtungsfaser (54) zu koppeln, wobei Eingänge der Optische-Kopplerkomponente (80) mit der Beleuchtungslichtquelle (61) bzw. der Behandlungslichtquelle (78) gekoppelt sind, und der Ausgang der Optische-Kopplerkomponente (80) mit der Kamerabeleuchtungsfaser (54) gekoppelt ist.

3. Raman-Spektroskopievorrichtung nach einem der Ansprüche 1 bis 2, wobei die Raman-Lichtquelle (40) und/oder die Beleuchtungslichtquelle (61) ausgelegt sind, um eine Intensität des erzeugten Lichts zu variieren.

4. Raman-Spektroskopievorrichtung nach Anspruch 3, wobei die Raman-Spektroskopievorrichtung (100) ferner eine Steuerung (84) zum Steuern der Intensität des durch die Raman-Lichtquelle (40) und/oder die Beleuchtungsquelle (61) erzeugten Lichts umfasst.

5. Raman-Spektroskopievorrichtung nach einem der Ansprüche 1 bis 4, wobei die Analysevorrichtung (12) ferner eine Optische-Beleuchtungskomponente (63) und eine Beleuchtungsquelle (61) zum Erzeugen von nichtmonochromatischem Licht umfasst, wobei die Optische-Beleuchtungskomponente (63) mit der zumindest einen Raman-Faser (27), der Raman-Lichtquelle (40) und der Beleuchtungsquelle (61) gekoppelt ist, wobei die optische Beleuchtungskomponente (63) ausgelegt ist, um das monochromatische Licht aus der Raman-Lichtquelle (40) und/oder das nichtmonochromatische Licht aus der Beleuchtungsquelle (61) selektiv oder permanent in die zumindest eine Raman-Faser (27) zu koppeln.

6. Raman-Spektroskopievorrichtung nach einem der Ansprüche 1 bis 5, wobei die Analysevorrichtung (12) ferner eine optische Detektionskomponente (76) umfasst, wobei die optische Detektionskomponente (76) mit der zumindest einen Raman-Faser (27), der Raman-Lichtquelle (40) und dem Spektrometer (72) gekoppelt ist, wobei die optische Detektionskomponente (76) ausgelegt ist, um das monochromatische Licht aus der Raman-Lichtquelle (40) in die zumindest eine Raman-Faser (27) zu leiten und das Licht aus der zumindest einen Raman-Faser (27) an das Spektrometer (72) zu leiten.

## Revendications

1. Appareil de spectroscopie Raman, comprenant
la sonde de spectroscopie Raman (10),
un dispositif d'analyse (12) incluant un spectromètre (72) et une source de lumière Raman (40) pour générer une lumière monochromatique,
une source de lumière de traitement (78) configurée pour générer une lumière présentant une intensité configurée pour modifier un tissu, et
un composant de couplage optique (80),
dans lequel la sonde de spectroscopie Raman (10) comprend
un corps allongé (16) présentant une extrémité distale (18) et une extrémité proximale (20),
au moins une fibre Raman (27) à l'intérieur du corps allongé (16) pour guider de la lumière entre l'extrémité proximale (20) et l'extrémité distale (18), et
une lumière d'instrument (26) à l'intérieur du corps allongé (16) et s'étendant entre l'extrémité distale (18) et l'extrémité proximale (20),
dans lequel la lumière d'instrument (26) est configurée pour recevoir un instrument allongé (32),
dans lequel la au moins une fibre Raman (27) est agencée à l'extérieur de la lumière d'instrument (26),
dans lequel la au moins une fibre Raman (27) est couplée au dispositif d'analyse (12), en particulier à la source de lumière Raman et/ou au spectromètre et
dans lequel le composant de couplage optique (80) est configuré pour coupler de manière sélective ou permanente la lumière provenant de la source de lumière Raman (40) et la lumière provenant de la source de lumière de traitement (78) dans la au moins une fibre Raman (27), dans lequel des entrées du composant de couplage optique (80) sont couplées respectivement à la source de lumière Raman (40) et à la source de lumière de traitement (78) et la sortie du composant de couplage optique (80) est couplée à la fibre Raman (27).

2. Appareil de spectroscopie Raman, comprenant
la sonde de spectroscopie Raman (10),
un dispositif d'analyse (12) incluant un spectromètre (72), une source de lumière Raman (40) pour générer une lumière monochromatique, et une source d'éclairage (61) pour générer une lumière non monochromatique,
une source de lumière de traitement (78) configurée pour générer une lumière présentant une intensité configurée pour modifier un tissu, et
un composant de couplage optique (80),
dans lequel la sonde de spectroscopie Raman (10) comprend
un corps allongé (16) présentant une extrémité distale (18) et une extrémité proximale (20),
au moins une fibre Raman (27) à l'intérieur du corps allongé (16) pour guider de la lumière entre l'extrémité proximale (20) et l'extrémité distale (18),
au moins une fibre d'éclairage de caméra (54) qui est configurée pour guider de la lumière à partir de l'extrémité proximale (18) vers l'extrémité distale (20) et
une lumière d'instrument (26) à l'intérieur du corps allongé (16) et s'étendant entre l'extrémité distale (18) et l'extrémité proximale (20),
dans lequel la lumière d'instrument (26) est configurée pour recevoir un instrument allongé (32),
dans lequel la au moins une fibre Raman (27) est agencée à l'extérieur de la lumière d'instrument (26),
dans lequel la au moins une fibre Raman (27) est couplée au dispositif d'analyse (12), en particulier à la source de lumière Raman et/ou au spectromètre et
dans lequel le composant de couplage optique (80) est configuré pour coupler de manière sélective ou permanente la lumière provenant de la source d'éclairage (61) et la lumière provenant de la source de lumière de traitement (78) dans la au moins une fibre d'éclairage de caméra (54), dans lequel des entrées du composant de couplage optique (80) sont couplées respectivement à la source d'éclairage (61) et à la source de lumière de traitement (78) et la sortie du composant de couplage optique (80) est couplée à la fibre d'éclairage de caméra (54).

3. Appareil de spectroscopie Raman selon l'une quelconque des revendications 1 à 2, dans lequel la source de lumière Raman (40) et/ou la source d'éclairage (61) sont configurées pour faire varier une intensité de la lumière générée.

4. Appareil de spectroscopie Raman selon la revendication 3, dans lequel l'appareil de spectroscopie Raman (100) comprend en outre un dispositif de commande (84) pour commander l'intensité de la lumière générée par la source de lumière Raman (40) et/ou la source d'éclairage (61).

5. Appareil de spectroscopie Raman selon l'une quelconque des revendications 1 à 4, dans lequel le dispositif d'analyse (12) inclut en outre un composant d'éclairage optique (63) et une source d'éclairage (61) pour générer une lumière non monochromatique, le composant d'éclairage optique (63) étant couplé à la au moins une fibre Raman (27), à la source de lumière Raman (40) et à la source d'éclairage (61), dans lequel le composant d'éclairage optique (63) est configuré pour coupler de manière sélective ou permanente la lumière monochromatique provenant de la source de lumière Raman (40) et/ou la lumière non monochromatique provenant de la source d'éclairage (61) dans la au moins une fibre Raman (27).

6. Appareil de spectroscopie Raman selon l'une quelconque des revendications 1 à 5, dans lequel le dispositif d'analyse (12) inclut en outre un composant de détection optique (76), le composant de détection optique (76) étant couplé à la au moins une fibre Raman (27), à la source de lumière Raman (40) et au spectromètre (72), dans lequel le composant de détection optique (76) est configuré pour acheminer la lumière monochromatique de la source de lumière Raman (40) dans la au moins une fibre Raman (27) et la lumière provenant de la au moins une fibre Raman (27) vers le spectromètre (72).
